# EUROPEAN PATENT SPECIFICATION

(11) **EP 1 090 020 B1**
(45) Date of publication and mention of the grant of the patent: **05.11.2003**
(21) Application number: 99929289.9
(22) Date of filing: 23.06.1999
(51) Int. Cl.: C07H 19/16, A61K 31/70, C07H 19/04

(54) **2-(PURIN-9-YL)-TETRAHYDROFURAN-3,4-DIOL DERIVATIVES**
2-(PURIN-9-YL)-TETRAHYDROFURAN-3,4-DIOL-DERIVATE
DERIVES DU 2-(PURIN-9-YL)-TETRAHYDROFURAN-3,4 DIOL

(30) Priority: 23.06.1998 GB 9813540
(43) Date of publication of application: 11.04.2001
(73) Proprietor: GLAXO GROUP LIMITED, Greenford, Middlesex UB6 ONN (GB)
(72) Inventor: ALLEN, David George, Stevenage, Hertfordshire SG1 2NY (GB); CHAN, Chuen, Stevenage, Hertfordshire SG1 2NY (GB); COOK, Caroline, Mary, Stevenage, Hertfordshire SG1 2NY (GB); COUSINS, Richard, Peter, Charles, Stevenage, Hertfordshire SG1 2NY (GB); COX, Brian, Stevenage, Hertfordshire SG1 2NY (GB); DYKE, Hazel Joan, Milton Road, Cambridge CB4 4WE (GB); ELLIS, Frank, Stevenage, Hertfordshire SG1 2NY (GB); GEDEN, Joanna Victoria, OSI Pharmaceuticals, Aston Science Park, Birmingham B7 4EJ (GB); HOBBS, Heather, Stevenage, Hertfordshire SG1 2NY (GB); KEELING, Suzanne, Elaine, Stevenage, Hertfordshire SG1 2NY (GB); REDGRAVE, Alison, Judith, Stevenage, Hertfordshire SG1 2NY (GB); SWANSON, Stephen, Stevenage, Hertfordshire SG1 2NY (GB); BAYS, David, Ware, Hertfordshire SG12 9PE (GB)
(74) Representative: Teuten, Andrew John
(86) International application number: EP9904271
(87) International publication number: WO99067263

(56) References cited:
- WO-A-93/22328
- WO-A-94/17090
- WO-A-96/02553
- WO-A-98/01459
- WO-A-98/28319
- WO-A-99/38877
- DE-A- 2 621 470
- US-A- 4 167 565
- KOBE J ET AL: "PREPARATION AND UTILITY OF 5-BETA-D-RIBOFURANOSYL-1H-TETRAZOLE AS A KEY SYNTHON FOR C-NUCLEOSIDE SYNTHESIS" NUCLEOSIDES & NUCLEOTIDES, vol. 13, no. 10, 1 January 1994 (1994-01-01), pages 2209-2244, XP002064181 ISSN: 0732-8311

## Description

This invention relates to new chemical compounds, processes for their preparation, pharmaceutical formulations containing them and their use in therapy.

Inflammation is a primary response to tissue injury or microbial invasion and is characterised by leukocyte adhesion to the endothelium, diapedesis and activation within the tissue. Leukocyte activation can result in the generation of toxic oxygen species (such as superoxide anion), and the release of granule products (such as peroxidases and proteases). Circulating leukocytes include neutrophils, eosinophils, basophils, monocytes and lymphocytes. Different forms of inflammation involve different types of infiltrating leukocytes, the particular profile being regulated by the profile of adhesion molecule, cytokine and chemotactic factor expression within the tissue.

The primary function of leukocytes is to defend the host from invading organisms such as bacteria and parasites. Once a tissue is injured or infected a series of events occurs which causes the local recruitment of leukocytes from the circulation into the affected tissue. Leukocyte recruitment is controlled to allow for the orderly destruction and phagocytosis of foreign or dead cells, followed by tissue repair and resolution of the inflammatory infiltrate. However in chronic inflammatory states, recruitment is often inappropriate, resolution is not adequately controlled and the inflammatory reaction causes tissue destruction.

There is evidence from both *in vitro* and *in vivo* studies to suggest that compounds active at the adenosine A2a receptor will have anti-inflammatory actions. The area has been reviewed by Cronstein (1994). Studies on isolated neutrophils show an A2 receptor-mediated inhibition of superoxide generation, degranulation, aggregation and adherence (Cronstein et al, 1983 and 1985; Burkey and Webster, 1993; Richter, 1992; Skubitz et al, 1988. When agents selective for the A2a receptor over the A2b receptor (eg CGS21680) have been used, the profile of inhibition appears consistent with an action on the A2a receptor subtype (Dianzani et al, 1994). Adenosine agonists may also down-regulate other classes of leucocytes (Elliot and Leonard, 1989; Peachell et al, 1989). Studies on whole animals have shown the anti-inflammatory effects of methotrexate to be mediated through adenosine and A2 receptor activation (Asako et al, 1993; Cronstein et al, 1993 and 1994). Adenosine itself, and compounds that raise circulating levels of adenosine also show anti-inflammatory effects *in vivo* (Green et al, 1991; Rosengren et al, 1995). In addition raised levels of circulating adenosine in man (as a result of adenosine deaminase deficiency) results in immunosuppression (Hirschom, 1993).

Certain substituted 4'-carboxamido and 4'-thioamido adenosine derivatives which are useful for the treatment of inflammatory diseases are described in International Patent Application Nos. WO94/17090, WO96/02553, WO96/02543 (Glaxo Group). Substituted 4'-carboxamidoadenosine derivatives useful in the treatment of dementia are described in AU 8771946 (Hoechst Japan). Substituted 4'-hydroxymethyl adenosine derivatives which are useful for the treatment of gastrointestinal motility disorders are described in EP-A-423776 and EP-A-423777 (Searle). Substituted 4'-hydroxymethyl adenosine derivatives which are useful as platelet aggregation inhibitors are described in BE-768925 (Takeda). 4'-Hydroxymethyl adenosine derivatives and 4'-esters thereof which are useful as anti-hypertensive agents or have other cardiovascular activity are described in US 4663313, EP 139358 and US 4767747 (Warner Lambert), US 4985409 (Nippon Zoki) and US 5043325 (Whitby Research). 4-Hydroxymethyladenosine derivatives useful in the treatment of autoimmune disorders are described in US 5106837 (Scripps Research Institute). 4'-Hydroxymethyladenosine derivatives useful as anti-allergic agents are described in US 4704381 (Boehringer Mannheim). Certain 4'-tetrazolylalkyl adenosine derivatives which are useful in the treatment of heart and circulatory disorders are generically described in DT-A-2621470 (Pharma-Waldhof). Other 4'-carboxamidoadenosine derivatives useful in the treatment of cardiovascular conditions are described in US 5219840, GB 2203149 and GB 2199036 (Sandoz), WO94/02497 (US Dept. Health), US 4968697 and EP 277917 (Ciba Geigy), US 5424297 (Univ. Virginia) and EP 232813 (Warner Lambert). Other 4'-carboxamidoadenosine derivatives lacking substitution on the purine ring in the 2-position are described in DT 2317770, DT 2213180, US 4167565, US 3864483 and US 3966917 (Abbott Labs), DT 2034785 (Boehringer Mannheim), JP 58174322 and JP 58167599 (Tanabe Seiyaku), WO92/05177 and US 5364862 (Rhone Poulenc Rorer), EP 66918 (Procter and Gamble), WO86/00310 (Nelson), EP 222330, US 4962194, WO88/03147 and WO88/03148 (Warner Lambert) and US 5219839, WO95/18817 and WO93/14102 (Lab UPSA). 4'-Hydroxymethyladenosine derivatives lacking substitution on the purine ring in the 2-position are described in WO95/11904 (Univ Florida). 4'-Substituted adenosine derivatives useful as adenosine kinase inhibitors are described in WO94/18215 (Gensia). Other 4'-halomethyl, methyl, thioalkylmethyl or alkoxymethyl adenosine derivatives are described in EP 161128 and EP 181129 (Warner Lambert) and US 3983104 (Schering). Other 4'-carboxamidoadenosine derivatives are described in US 7577528 (NIH), WO91/13082 (Whitby Research) and WO95/02604 (US Dept Health).

Certain tetrazole containing deoxynucleotides which were found to lack antiinfective activity are described in Baker et al (1974) Tetrahedron 30, 2939-2942. Other tetrazole containing adenosine derivatives which show activity as platelet aggregation inhibitors are described in Mester and Mester (1972) Pathologie-Biologie, 20 (Suppl) 11-14. Certain nitrile containing ribose derivatives are described in Schmidt et al (1974) Liebigs. Ann. Chem. 1856-1863.

Other publications include: WO 98/16539 (Novo Nordisk A/S) which describes adenosine derivatives for the treatment of myocardial and cerebral ischaemia and epilepsy; WO 98/01426 (Rhone-Poulenc Rorer Pharmaceuticals Inc.) which relates to adenosine derivatives possessing antihypertensive, cardioprotective, anti-ischaemic and antilipolytic properties; and WO 98/01459 (Novo Nordisk A/S) which describes N,9-disubstituted adenine derivatives which are substituted in the 4' position by unsubstituted oxazolyl or isoxazolyl and the use of such compounds for the treatment of disorders involving cytokines in humans. WO 98/28319 (Glaxo. Group Limited) was published subsequent to the earliest priority date of this application and describes 4'-substituted tetrazole 2-(purin-9-yl)-tetrahydrofuran-3,4-diol derivatives;

We have now found a novel group of compounds with broad anti-inflammatory properties which inhibit leukocyte recruitment and activation and which are agonists of the adenosine 2a receptor. The compounds are therefore of potential therapeutic benefit in providing protection from leukocyte-induced tissue damage in diseases where leukocytes are implicated at the site of inflammation. The compounds of the invention may also represent a safer alternative to corticosteroids in the treatment of inflammatory diseases, whose uses are severely limited by their side-effect profiles.

More particularly, the compounds of this invention may show an improved profile over known A2a-selective agonists in that they generally lack agonist activity at the human A3 receptor. This profile can be considered of benefit as A3 receptors are also found on leucocytes (eg eosinophil) and other inflammatory cells (eg mast cell) and activation of these receptors may have pro-inflammatory effects (Kohno et al, 1996; Van Schaick et al 1996). It is even considered that the bronchoconstrictor effects of adenosine in asthmatics may be mediated via the adenosine A3 receptor (Kohno et al, 1996).

Thus, according to the invention we provide compounds of formula (I): 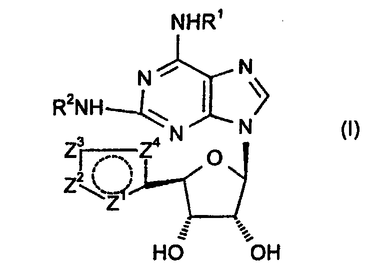 wherein R¹ and R² independently represent a group selected from:
(i) C₃₋₈cycloalkyl-;
(ii) hydrogen;
(iii) aryl₂CHCH₂-;
(iv) C₃₋₈cycloalkylC₁₋₆alkyl-;
(v) C₁₋₈alkyl-;
(vi) arylC₁₋₆alkyl-;
(vii) R⁴R⁵N-C₁₋₆alkyl-;
(viii) C₁₋₆alkyl-CH(CH₂OH)-;
(ix) arylC₁₋₅alkyl-CH(CH₂OH)-;
(x) arylC₁₋₅alkyl-C(CH₂OH)₂-;
(xi) C₃₋₈cycloalkyl independently substituted by one or more (e.g. 1, 2 or 3) -(CH₂)ₚR⁶ groups;
(xii) H₂NC(=NH)NHC₁₋₆alkyl-;
(xiii) a group of formula 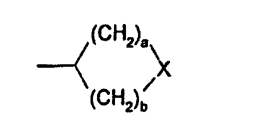 or such a group in which one methylene carbon atom adjacent to X, or both if such exist, is substituted by methyl;
(xiv) -C₁₋₆alkyl-OH;
(xv) -C₁₋₈haloalkyl;
(xvi) a group of formula 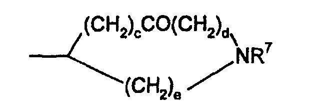
(xvii) aryl; and
(xviii) -(CH₂)_{f}SO₂NH_{g}(C₁₋₄alkyl-)_{2-g} or -(CH₂)_{f}SO₂NH_{g}(arylC₁₋₄alkyl-)_{2-g} where f is 2 or 3 and g is an integer 0 to 2;
Z¹, Z², Z³ and Z⁴ together with the carbon atom form a 5-membered heterocyclic aromatic ring;
R⁴ and R⁵ independently represent hydrogen, C₁₋₆alkyl, aryl, arylC₁₋₆alkyl- or NR⁴R⁵ together may represent pyridinyl, pyrrolidinyl, piperidinyl, morpholinyl, azetidinyl, azepinyl, piperazinyl or N-C₁₋₆alkylpiperazinyl;
R⁶ represents OH, NH₂, NHCOCH₃ or halogen;
R⁷ represents hydrogen, C₁₋₆alkyl, -C₁₋₆alkylaryl or -COC₁₋₆alkyl;
X represents NR⁷, O, S, SO or SO₂;
p represents 0 or 1;
a and b independently represent an integer 0 to 4 provided that a + b is in the range 3 to 5;
c, d and e independently represent an integer 0 to 3 provided that c + d + e is in the range 2 to 3;
and salts and solvates thereof.

Z¹, Z², Z³ and Z⁴ will independently represent C, N, O or S and, in the case of C and N, together with a sufficient number of hydrogen atoms to provide the ring with aromatic character. At least one of Z¹, Z², Z³ and Z⁴ will represent a heteroatom. Preferably at least one of Z¹, Z², Z³ and Z⁴ will represent a nitrogen atom. More preferably at least one of Z¹, Z², Z³ and Z⁴ will represent a nitrogen atom, two of the remainder independently will represent C or N and the fourth will represent C, N or O. In each case sufficient hydrogen atoms will be provided to give the ring aromatic character. However, we prefer that Z¹, Z², Z³ and Z⁴ do not all represent nitrogen

References to C_{x-y}alkyl include references to an aliphatic hydrocarbon grouping containing x to y carbon atoms which may be straight chain or branched and may be saturated or unsaturated. References to alkoxy may also be interpreted similarly.

References to aryl include references to mono- and bicyclic carbocyclic aromatic rings (e.g. phenyl, naphthyl) and heterocyclic aromatic rings, for example containing 1-3 hetero atoms selected from N, O and S (e.g. pyridinyl, pyrimidinyl, thiophenyl, imidazolyl, quinolinyl, furanyl, pyrrolyl, oxazolyl) all of which may be optionally substituted, e.g. by C₁₋₆alkyl, halogen, hydroxy, nitro, C₁₋₆ alkoxy, cyano, amino, SO₂NH₂ or -CH₂OH.

Examples of C₃₋₈cycloalkyl for R¹ and R² include monocyclic alkyl groups (e.g. cyclopentyl, cyclohexyl) and bicyclic alkyl groups (e.g. norbornyl such as exo-norbom-2-yl).
Examples of (aryl)₂CHCH₂- for R¹ and R² include Ph₂CHCH₂- or such a group in which one or both phenyl moieties is substituted, e.g. by halogen or C₁₋₄alkyl.
Examples of C₃₋₈cycloalkylC₁₋₆alkyl- for R¹ and R² include ethylcyclohexyl.

Examples of C₁₋₈alkyl for R¹ and R² include -(CH₂)₂C(Me)₃, -CH(Et)₂ and CH₂=C(Me)CH₂CH₂-.
Examples of arylC₁₋₆alkyl- for R¹ and R² include -(CH₂)₂Ph, -CH₂Ph or either in which Ph is substituted (one or more times) by halogen (e.g. iodine), amino, methoxy, hydroxy, -CH₂OH or SO₂NH₂; -(CH₂)₂ pyridinyl (e.g. -(CH₂)₂pyridin-2-yl) optionally substituted by amino; (CH₂)₂imidazolyl (e.g. 1H-imidazol-4-yl) or this group in which imidazoyl is N-substituted by C₁₋₆alkyl (especially methyl).
Examples of R⁴R⁵N-C₁₋₆alkyl- for R¹ and R² include ethyl-piperidin-1-yl, ethyl-pyrrolidin-1-yl, ethyl-morpholin-1-yl, -(CH₂)₂NH(pyridin-2-yl) and -(CH₂)₂NH₂.
Examples of C₁₋₆alkyl-CH(CH₂OH)- for R¹ and R² include Me₂CHCH(CH₂OH)-.
Examples of arylC₁₋₅alkyl-CH(CH₂OH)- for R¹ and R² include PhCH₂CH(CH₂OH)-particularly 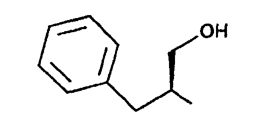 Examples of arylC₁₋₅alkyl-C(CH₂OH)₂- for R¹ and R² include PhCH₂C(CH₂OH)₂-.
Examples of C₃₋₈ cycloalkyl independently substituted by one or more -(CH₂)ₚR⁶ groups (eg 1, 2 or 3 such groups) for R¹ and R² include 2-hydroxy-cyclopentyl and 4-aminocyclohexyl (especially trans-4-amino-cyclohexyl).
Examples of H₂NC(=NH)NHC₁₋₈alkyl for R¹ and R² include H₂NC(=NH)NH(CH₂)₂-Examples of groups of formula 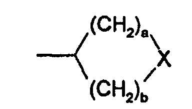 for R¹ and R² include pyrrolidin-3-yl, piperidin-3-yl, piperidin-4-yl, tetrahydro-1,1-dioxide thiophen-3-yl, tetrahydropyran-4-yl, tetrahydrothiopyran-4-yl and 1,1-dioxo-hexahydro-1.lamda.6-thiopyran-4-yl, or a derivative in which the ring nitrogen is substituted by C₁₋₆alkyl (e.g. methyl), C₁₋₆alkylacyl (e.g. acetyl), arylC₁₋₆ alkyl- (e.g. benzyl).

Examples of -C₁₋₆alkyl-OH groups for R¹ and R² include -CH₂CH₂OH and -CH(CH₂OH)CH(CH₃)₂.
Examples of C₁₋₈haloalkyl for R¹ and R² include -CH₂CH₂Cl and (CH₃)₂ClC(CH₂)₃-.
Examples of groups of formula 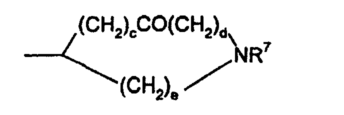 for R¹ and R² include 2-oxopyrrolidin-4-yl, 2-oxopyrrolidin-3-yl or a derivative in which the ring nitrogen is substituted by C₁₋₆alkyl (e.g. methyl) or benzyl.
Examples of aryl for R¹ and R² include phenyl optionally substituted by halogen (e.g. fluorine, especially 4-fluorine).
An example of a -(CH₂)_{f}SO₂NH_{g}(C₁₋₄alkyl)_{2-g} group for R¹ and R² is -(CH₂)₂SO₂NHMe, and an example of a -(CH₂)_{f}SO₂NH_{g}(arylC₁₋₄alkyl)_{2-g} group for R¹ and R² is -(CH₂)₂SO₂NHCH₂Ph.
An example of C₁₋₆alkyl for R⁷ is methyl, an example of C₁₋₈alkylaryl for R⁷ is benzyl, and an example of -COC₁₋₆alkyl for R⁷ is acetyl.

We prefer that R¹ and R² do not both represent hydrogen.
We prefer R¹ to represent aryl₂CHCH₂-, C₁₋₈alkyl-, hydrogen or arylC₁₋₆alkyl.
We prefer R² to represent ethyl-piperidin-1-yl, PhCH₂CH(CH₂OH)-, - CH(CH₂OH)CH(CH₃)₂, trans-4-amino-cyclohexyl, 2-(1-methyl-1H-imidazoyl-4-yl)CH₂CH₂-, pyrrolidin-3-yl, ethyl-pyridin-2-yl, H₂NC(=NH)NH(CH₂)₂-, or cyclopentyl.
We prefer R⁴ and R⁵ independently to represent hydrogen or aryl or NR⁴R⁵ together to represent pyrrolidinyl, piperidinyl, morpholinyl, azetidinyl, azepinyl, piperazinyl or N-methylpiperazinyl.
We prefer that p represents 0. We prefer that R⁶ represents OH or NH₂.
We prefer that a represents 2 and that b represents 1 or 2. We prefer X to represent NR⁷ (e.g. NH), O, S or SO₂, particularly O, S or NH.

We prefer that c represents 0, and either that d represents 1 and e represents 1 or d represents 0 and e represents 2. We prefer that R⁷ represents hydrogen.

We particularly prefer R¹ to represent Ph₂CHCH₂-, hydrogen, CH(Et₂) or PhCH₂CH₂-, especially Ph₂CHCH₂.
We particularly prefer R² to represent -CH(CH₂OH)CH(CH₃)₂ or 2-(1-methyl-1H-imidazoyl-4-yl)CH₂CH₂-.

We prefer that the moiety 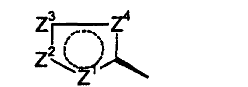 represents one of the following groups: 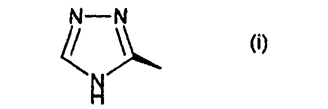 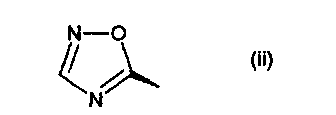 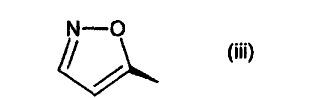 wherein the group: 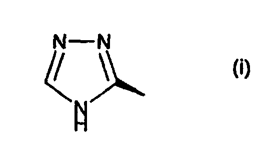 is particularly preferred.

The representation of formula (I) indicates the absolute stereochemistry. When sidechains contain chiral centres the invention extends to mixtures of enantiomers (including racemic mixtures) and diastereoisomers as well as individual enantiomers. Generally it is preferred to use a compound of formula (I) in the form of a purified single enantiomer.

Particularly preferred compounds herein have formula (la) in which R^{2a}, R^{1a} and the Z¹-Z⁴ comprising ring are as defined in the table below:

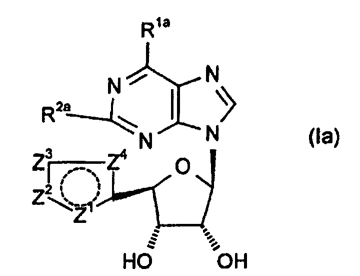

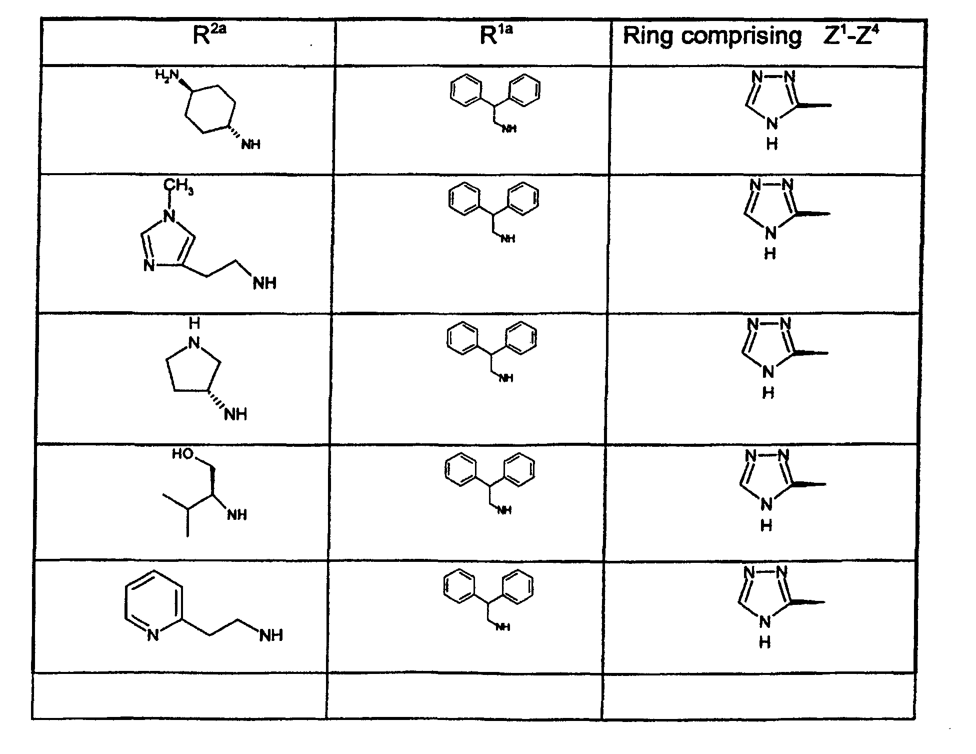

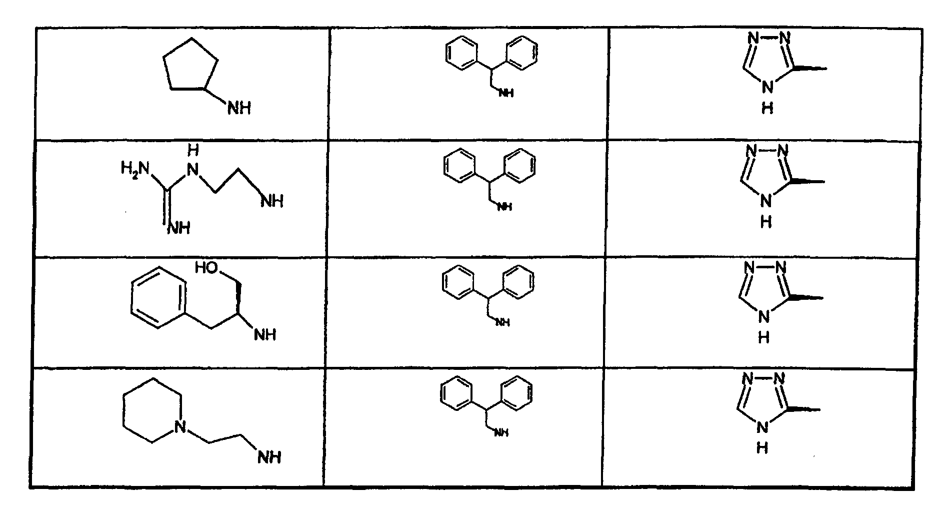

We also provide a first process for the preparation of compounds of formula (I) including the step of reacting a compound of formula (II) 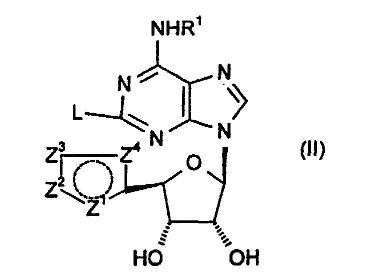 wherein L represents a leaving group, e.g. halogen, particularly chlorine; or a protected derivative thereof with a compound of formula R²NH₂ or a protected derivative thereof. Said reaction will generally involve heating the reagents to a temperature of 50°C-150°C in the presence of an inert solvent such as DMSO. The compound of formula (II) may be used in a form which the two hydroxyl groups are protected e.g. with acetonide or acetyl groups. Compounds of formula R²NH₂ are either known or may be prepared by conventional methods known *per se*.

Compounds of formula (II) or a protected derivative thereof may be prepared by reacting a compound of formula (III) 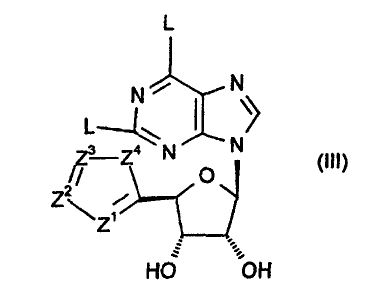 or a protected derivative thereof with a compound of formula R¹NH₂. This reaction will preferably be performed in the presence of a base such as an amine base (e.g. diisopropyl ethylamine in a solvent such as an alcohol (e.g. isopropanol) at elevated temperature (e.g. 50°C).

The compound of formula (III) wherein L represents chlorine or a protective derivative thereof may be prepared by reacting a compound of formula (IV) 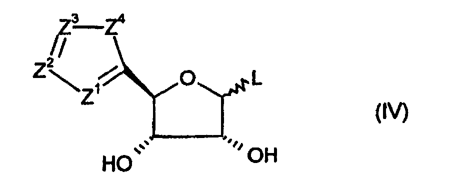 wherein L represents a leaving group, or a protected derivative thereof with 2,6,dichloropurine.

We prefer to use the compound of formula (IV) when the ribose 2- and 3-hydroxyl groups are protected for example by acetyl. Leaving group L may represent OH but will preferably represent C₁₋₆alkoxy (e.g. methoxy or ethoxy) an ester moiety (e.g. acetyloxy or benzoyloxy) or halogen. The preferred group L is acetyloxy. The reaction may be formed by combining the reactants in an inert solvent such as MeCN in the presence of a Lewis Acid (e.g. TMSOTf) and DBU.

The compounds of formula (IV) or a protected derivatives thereof may be prepared from a compound of formula (V) 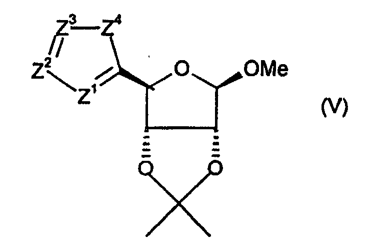 by treating the compound of formula (V) with trifluoroacetic acid in water followed by acetic anhydride in a solvent such as pyridine, DMAP, Et₃N, DCM or a combination of these.

Compounds of formula (IV) in which L represents halogen may be prepared for the corresponding 1'-alcohol or a 1'-ester such as the acetate. Reaction will generally occur on treatment with anhydous HCI or HBr. 1'-lodides may be prepared directly on treatment with trimethylsilyliodide and 1'-fluorides may be prepared on treatment with DAST. An inert solvent, e.g. diethylether, DCM, THF or CCI₄ will generally be suitable.

Compounds of formula (V) may be prepared from D-ribose using methods analogous to those described at Scheme 1 of PCT Application No. PCT/EP97/07197, wherein the heterocyclic ring formation is achieved by conventional methods known *per se*.

We also provide a second process for the preparation of compounds of formula (II) including the step of reacting a compound of formula (VI) 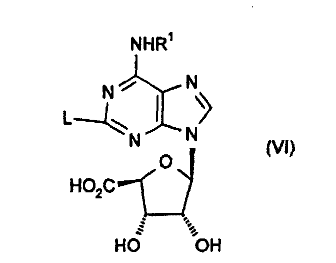 with reagents to enable formation of the appropriate heterocyclic ring (comprising Z¹, Z², Z³, and Z⁴) using conventional heterocyclic ring formation methods known *per se.* The compound of formula (VI) wherein R¹ represents Ph₂CHCH₂ and L represents chlorine is known and described at preparation 4. of PCT Patent Application No. WO94/17090. Other compounds of formula (VI) may be prepared by analogous or conventional methods.

This second process is particularly suitable for making compounds herein where the heterocyclic ring is a triazolyl. A preferred reaction scheme of this type comprises: 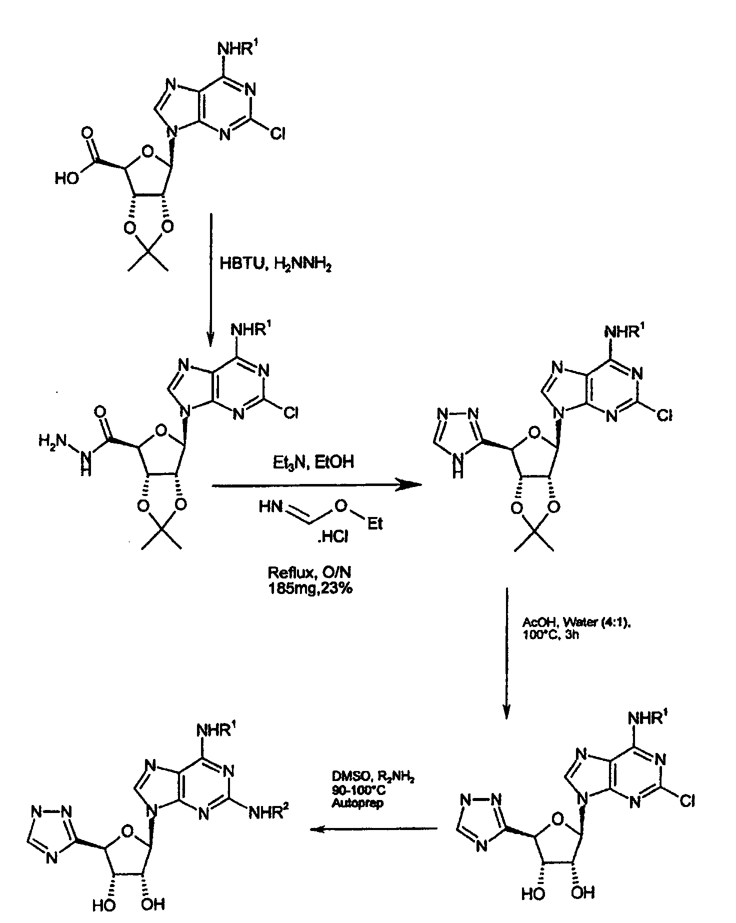 In a third process, where the moiety: 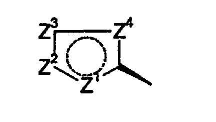 is represented by: 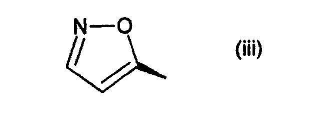 the compounds of formula (I) may be obtained by dehalogenating a compound of formula (VII):

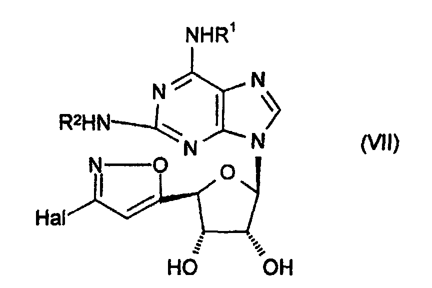

The dehalogenation can, for example, be achieved by known catalytic hydrogenation processes.

Compounds of formula (VII) wherein Hal represents Br may be prepared according to the following scheme:

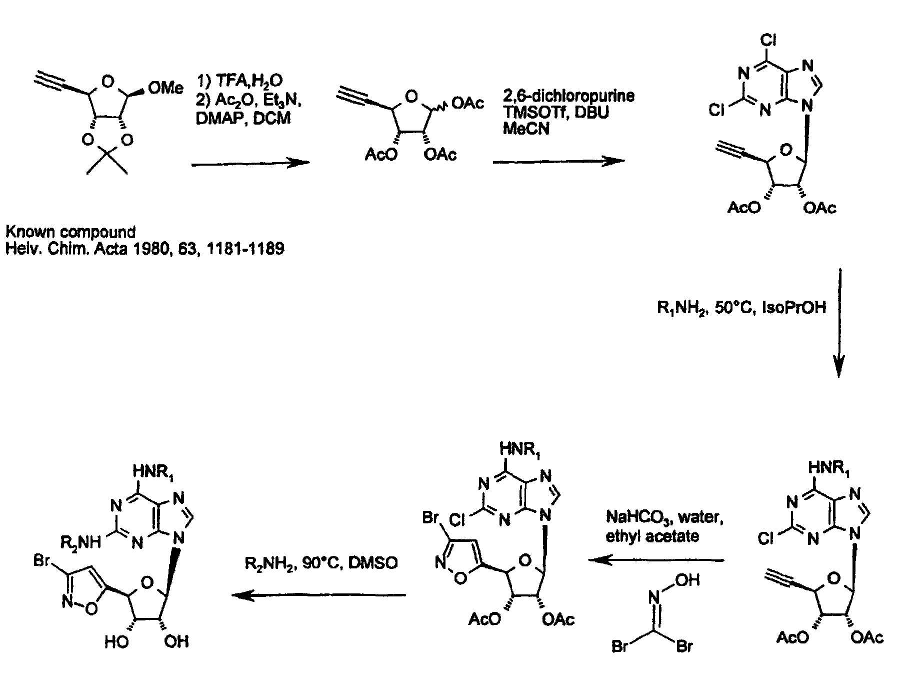

Examples of protecting groups and the means for their removal can be found in T W Greene "Protective Groups in Organic Synthesis" (J Wiley and Sons, 1991). Suitable hydroxyl protecting groups include alkyl (e.g. methyl), acetal (e.g. acetonide) and acyl (e.g. acetyl or benzoyl) which may be removed by hydrolysis, and arylalkyl (e.g. benzyl) which may be removed by catalytic hydrogenolysis. Suitable amine protecting groups include sulphonyl (e.g. tosyl), acyl e.g. benzyloxycarbonyl or t-butoxycarbonyl) and arylalkyl (e.g. benzyl) which may be removed by hydrolysis or hydrogenolysis as appropriate.

Suitable salts of the compounds of formula (I) include physiologically acceptable salts such as acid addition salts derived from inorganic or organic acids, for example hydrochlorides, hydrobromides, sulphates, phosphates, acetates, benzoates, citrates, succinates, lactates, tartrates, fumarates, maleates, 1-hydroxy-2-naphthoates, methanesulphonates, and if appropriate, inorganic base salts such as alkali metal salts, for example sodium salts. Other salts of the compounds of formula (I) include salts which are not physiologically acceptable but may be useful in the preparation of compounds of formula (I) and physiologically acceptable salts thereof. Examples of such salts include trifluoroacetates and formates.

Examples of suitable solvates of the compounds of formula (I) include hydrates. Acid-addition salts of compounds of formula (I) may be obtained by treating a free-base of formula (I) with an appropriate acid.

The potential for compounds of formula (I) to inhibit leukocyte function may be demonstrated, for example, by their ability to inhibit superoxide (O₂⁻) generation from neutrophils stimulated with chemoattractants such as N-formylmethionyl-leucyl-phenylalanine (fMLP). Accordingly, compounds of formula (I) are of potential therapeutic benefit in providing protection from leukocyte-induced tissue damage in diseases where leukocytes are implicated at the site of inflammation.

Examples of disease states in which the compounds of the invention have potentially beneficial anti-inflammatory effects include diseases of the respiratory tract such as adult respiratory distress syndrome (ARDS), bronchitis (including chronic bronchitis), cystic fibrosis, asthma (including allergen-induced asthmatic reactions), emphysema, rhinitis and septic shock. Other relevant disease states include diseases of the gastrointestinal tract such as intestinal inflammatory diseases including inflammatory bowel disease (e.g. Crohn's disease or ulcerative colitis), Helicobacter-pylori induced gastritis and intestinal inflammatory diseases secondary to radiation exposure or allergen exposure, and non-steroidal anti-inflammatory drug-induced gastropathy. Furthermore, compounds of the invention may be used to treat skin diseases such as psoriasis, allergic dermatitis and hypersensitivity reactions and diseases of the central nervous system which have an inflammatory component e.g. Alzheimer's disease and multiple sclerosis.

Further examples of disease states in which compounds of the invention have potentially beneficial effects include cardiac conditions such as peripheral vascular disease, post-ischaemic reperfusion injury and idiopathic hypereosinophilic syndrome.

Compounds of the invention which inhibit lymphocyte function may be useful as immunosuppressive agents and so have use in the treatment of auto-immune diseases such as rheumatoid arthritis and diabetes.

Compounds of the invention may also be useful in inhibiting metastasis.

It will be appreciated by those skilled in the art that reference herein to treatment extends to prophylaxis as well as the treatment of established conditions.

As mentioned above, compounds of formula (I) are useful in human or veterinary medicine, in particular as anti-inflammatory agents.

There is thus provided as a further aspect of the invention a compound of formula (I) or a physiologically acceptable salt or solvate thereof for use in human or veterinary medicine, particularly in the treatment of patients with inflammatory conditions who are susceptible to leukocyte-induced tissue damage.
According to another aspect of the invention, there is provided the use of a compound of formula (I) or a physiologically acceptable salt or solvate thereof for the manufacture of a medicament for the treatment of patients with inflammatory conditions who are susceptible to leukocyte-induced tissue damage.

The compounds according to the invention may be formulated for administration in any convenient way, and the invention therefore also includes within its scope pharmaceutical compositions for use in anti-inflammatory therapy, comprising a compound of formula (I) or a physiologically acceptable salt or solvate thereof together, if desirable, with one or more physiologically acceptable carriers or excipients.

There is also provided a process for preparing such a pharmaceutical formulation which comprises mixing the ingredients.

The compounds according to the invention may, for example, be formulated for oral, buccal, parenteral, topical or rectal administration, preferably for parenteral or topical (e.g. by aerosol) administration.

Tablets and capsules for oral administration may contain conventional excipients such as binding agents, for example syrup, acacia, gelatin, sorbitol, tragacanth, mucilage of starch, cellulose or polyvinyl pyrrolidone; fillers, for example, lactose, microcrystalline cellulose, sugar, maize- starch, calcium phosphate or sorbitol; lubricants, for example, magnesium stearate, stearic acid, talc, polyethylene glycol or silica; disintegrants, for example, potato starch, croscarmellose sodium or sodium starch glycollate; or wetting agents such as sodium lauryl sulphate. The tablets may be coated according to methods well known in the art. Oral liquid preparations may be in the form of, for example, aqueous or oily suspensions, solutions, emulsions, syrups or elixirs, or may be presented as a dry product for constitution with water or other suitable vehicle before use. Such liquid preparations may contain conventional additives such as suspending agents, for example, sorbitol syrup, methyl cellulose, glucose/sugar syrup, gelatin, hydroxymethyl cellulose, carboxymethyl cellulose, aluminium stearate gel or hydrogenated edible fats; emulsifying agents, for example, lecithin, sorbitan mono-oleate or acacia; non-aqueous vehicles (which may include edible oils), for example almond oil, fractionated coconut oil, oily esters, propylene glycol or ethyl alcohol; or preservatives, for example, methyl or propyl p- hydroxybenzoates or sorbic acid. The preparations may also contain buffer salts, flavouring, colouring and/or sweetening agents (e.g. mannitol) as appropriate.

For buccal administration the compositions may take the form of tablets or lozenges formulated in conventional manner.

The compounds may also be formulated as suppositories, e.g. containing conventional suppository bases such as cocoa butter or other glycerides.

The compounds according to the invention may also be formulated for parenteral administration by bolus injection or continuous infusion and may be presented in unit dose form, for instance as ampoules, vials, small volume infusions or pre-filled syringes, or in multi-dose containers with an added preservative. The compositions may take such forms as solutions, suspensions, or emulsions in aqueous or non-aqueous vehicles, and may contain formulatory agents such as anti-oxidants, buffers, antimicrobial agents and/or tonicity adjusting agents. Alternatively, the active ingredient may be in powder form for constitution with a suitable vehicle, e.g. sterile, pyrogen-free water, before use. The dry solid presentation may be prepared by filling a sterile powder aseptically into individual sterile containers or by filling a sterile solution aseptically into each container and freeze-drying.

By topical administration as used herein, we include administration by insufflation and inhalation. Examples of various types of preparation for topical administration include ointments, creams, lotions, powders, pessaries, sprays, aerosols, capsules or cartridges for use in an inhaler or insufflator, solutions for nebulisation or drops (e.g. eye or nose drops).

Ointments and creams may, for example, be formulated with an aqueous or oily base with the addition of suitable thickening and/or gelling agents and/or solvents. Such bases may thus, for example, include water and/or an oil such as liquid paraffin or a vegetable oil such as arachis oil or castor oil or a solvent such as a polyethylene glycol. Thickening agents which may be used include soft paraffin, aluminium stearate, cetostearyl alcohol, polyethylene glycols, microcrystalline wax and beeswax.

Lotions may be formulated with an aqueous or oily base and will in general also contain one or more emulsifying agents, stabilising agents, dispersing agents, suspending agents or thickening agents.

Powders for external application may be formed with the aid of any suitable powder base, for example, talc, lactose or starch. Drops may be formulated with an aqueous or non-aqueous base also comprising one or more dispersing agents, solubilising agents or suspending agents.

Spray compositions may be formulated, for example, as aqueous solutions or suspensions or as aerosols delivered from pressurised packs, with the use of a suitable propellant, e.g. dichlorodifluoromethane, trichlorofluoromethane, dichlorotetra-fluoroethane, 1,1,1,2,3,3,3-heptafluoropropane, 1,1,1,2-tetrafluoroethane, carbon dioxide or other suitable gas.

Intranasal sprays may be formulated with aqueous or non-aqueous vehicles with the addition of agents such as thickening agents, buffer salts or acid or alkali to adjust the pH, isotonicity adjusting agents or anti-oxidants.

Capsules and cartridges of for example gelatin, or blisters of for example laminated aluminium foil, for use in an inhaler or insufflator may be formulated containing a powder mix of a compound of the invention and a suitable powder base such as lactose or starch.

Solutions for inhalation by nebulation may be formulated with an aqueous vehicle with the addition of agents such as acid or alkali, buffer salts, isotonicity adjusting agents or antimicrobials. They may be sterilised by filtration or heating in an autoclave, or presented as a non-sterile product.

The pharmaceutical compositions according to the invention may also be used in combination with other therapeutic agents, for example anti-inflammatory agents (such as corticosteroids (e.g. fluticasone propionate, beclomethasone dipropionate, mometasone furoate, triamcinolone acetonide or budesonide) or NSAIDs (eg sodium cromoglycate)) or beta adrenergic agents (such as salmeterol, salbutamol, formoterol, fenoterol or terbutaline and salts thereof) or antiinfective agents (eg antibiotics, antivirals).

The invention thus provides, in a further aspect, a combination comprising a compound of formula (I) or a physiologically acceptable salt or solvate thereof together with another therapeutically active agent, for example an anti-inflammatory agent such as a corticosteroid or NSAID.

The combination referred to above may conveniently be presented for use in the form of a pharmaceutical formulation and thus pharmaceutical formulations comprising a combination as defined above together with a pharmaceutically acceptable carrier thereof represent a further aspect of the invention.

The individual components of such combinations may be administered either sequentially or simultaneously in separate or combined pharmaceutical formulations. Appropriate doses of known therapeutic agents will be readily appreciated by those skilled in the art.

Compounds of the invention may conveniently be administered in amounts of, for example, 0.01 to 500mg/kg body weight, preferably 0.01 to 100mg/kg body weight, 1 to 4 times daily. The precise dose will of course depend on the age and condition of the patient and the particular route of administration chosen.

Certain intermediate compounds described herein are new and these are also provided as an aspect of the invention.

The compounds of the invention have the advantage that they may be more efficacious, show greater selectivity, have fewer side effects, have a longer duration of action, be more bioavailable by the preferred route, show less systemic activity when administered by inhalation or have other more desirable properties than similar known compounds.

In particular the compounds of the invention have the advantage that they may show greater selectivity for the adenosine 2a receptor subtype over other adenosine receptor subtypes (especially the A1 and A3 receptor subtypes) than hitherto known compounds.

Compounds of the invention were tested for in vitro and in vivo biological activity in accordance with the following screens:
(1) Agonist activity against adenosine 2a, adenosine 1 and adenosine 3 receptor subtypes.
   Agonist selectivity of compounds against other human adenosine receptors was determined using Chinese hamster ovary (CHO) cells transfected with the gene for the relevant human adenosine receptor following a method based on that of Castanon and Spevak, 1994 . The CHO cells were also transfected with cyclic AMP response elements promoting the gene for secreted placental alkaline phosphatase (SPAP) (Wood, 1995). The effect of test compounds was determined by their effects on basal levels of CAMP (A2a) or on forskolin-enhanced cAMP (A1 and A3) as reflected by changes in levels of SPAP. EC₅₀ values for compounds were then determined as a ratio to that of the non-selective agonist N-ethyl carboxamide adenosine (NECA).

### References:

Asako H, Wolf, RE, Granger, DN (1993), Gastroenterology 104, pp 31-37;
Bedford CD, Howd RA, Dailey OD, Miller A, Nolen HW III, Kenley RA, Kern JR, Winterle JS, (1986), J. Med. Chem. 29, pp2174-2183;
Burkey TH, Webster, RO, (1993), Biochem. Biophys Acta 1175, pp 312-318;
Castanon MJ, Spevak W, (1994), Biochem. Biophys Res. Commun. 198, pp 626-631;
Cronstein BN, Kramer SB, Weissmann G, Hirschhom R, (1983), Trans. Assoc. Am. Physicians 96, pp 384-91;
Cronstein BN, Kramer SB, Rosenstein ED, Weissmann G, Hirschhom R, (1985), Ann N.Y. Acad. Sci. 451, pp 291-301;
Cronstein BN, Naime D, Ostad E, (1993), J. Clin. Invest 92, pp 2675-82;
Cronstein BN, Naime D, Ostad E, (1994), Adv. Exp. Med. Biol., 370, pp 411-6;
Cronstein BN, (1994), J. Appl. Physiol. 76, pp 5-13;
Dianzani C, Brunelleschi S, Viano I, Fantozzi R, (1994), Eur. J. Pharmacol 263, pp 223-226;
Elliot KRF, Leonard EJ, (1989), FEBS Letters 254, pp 94-98;
Flora KP, van't Riet B, Wampler GL, (1978), Cancer Research, 38, pp1291-1295;
Green PG, Basbaum Al, Helms C, Levine JD, (1991), Proc. Natl. Acad Sci. 88, pp 4162-4165;
Hirschorn R, (1993), Pediatr. Res 33, pp S35-41;
Kohno Y; Xiao-duo J; Mawhorter SD; Koshiba M; Jacobson KA. (1996).Blood 88 p3569-3574.
Peachell PT, Lichtenstein LM, Schleimer RP, (1989), Biochem Pharmacol 38, pp 1717-1725;
Richter J, (1992), J. Leukocyte Biol. 51, pp 270-275;
Rosengren S, Bong GW, Firestein GS, (1995), J. Immunol. 154, pp 5444-5451;
Sanjar S, McCabe PJ, Fattah D, Humbles AA, Pole SM, (1992), Am. Rev. Respir. Dis. 145, A40;
Skubitz KM, Wickman NW, Hammerschmidt DE, (1988), Blood 72, pp 29-33
Van Schaick EA; Jacobson KA; Kim HO; Ijzerman AP; Danhof M. (1996) Eur J Pharmacol 308 p311-314.
Wood KV. (1995) Curr Opinion Biotechnology 6 p50-58.

Throughout the specification and the claims which follow, unless the context requires otherwise, the word 'comprise', and variations such as 'comprises' and 'comprising', will be understood to imply the inclusion of a stated integer or step or group of integers but not to the exclusion of any other integer or step or group of integers or steps.

### The invention is illustrated by the following Examples:

### Examples

### General experimental details

Where products were purified by column chromatography, 'flash silica' refers to silica gel for chromatography, 0.040 to 0.063mm mesh (e.g. Merck Art 9385), where column elution was accelerated by an applied pressure of nitrogen at up to 5 p.s.i. Where thin layer chromatography (TLC) has been used it refers to silica gel TLC using 5 x 10 cm silica gel 60 F₂₅₄ plates (e.g. Merck Art 5719). Where products were purified by preparative HPLC, this was carried out on a C18-reverse-phase column (1" Dynamax), eluting with a gradient of acetonitrile (containing 0.1% trifluoroacetic acid) in water (containing 0.1% trifluoroacetic acid) and the compounds isolated as their trifluoroacetate salts unless otherwise specified.

### Standard Automated Preparative HPLC column, conditions & eluent

Automated preparative high performance liquid chromatography (autoprep. HPLC) was carried out using a Supelco ABZ+ 5µm 100mmx22mm i.d. column eluted with a mixture of solvents consisting of i) 0.1% formic acid in water and ii) 0.05% formic acid in acetonitrile, the eluent being expressed as the percentage of ii) in the solvent mixture, at a flow rate of 4ml per minute. Unless otherwise stated the eluent was used as a gradient of 5-95 % over 20 minutes.

### LC/MS System

The Liquid Chromatography Mass Spectroscopy (LC/MS) systems used:
LC/MS System A - A Supelco ABZ+, 3.3cm x 4.6mm i.d. column eluting with solvents: A - 0.1%v/v formic acid + 0.077% w/v ammonium acetate in water, and B - 95:5 acetonitrile:water + 0.05% v/v formic acid. The following gradient protocol was used: 100% A for 0.7 mins; A+B mixtures, gradient profile 0 - 100% B over 3.5mins; hold at 100% B for 3.5mins; return to 0% B over 0.3mins. Positive and negative electrospray ionization was employed.
LC/MS System B - A Supelco ABZ+, 5cm x 2.1mm i.d. column eluting with solvents: A - 0.1%v/v formic acid + 0.077% w/v ammonium acetate in water, and B - 95:5 acetonitrile:water + 0.05% v/v formic acid. The following gradient protocol was used: 0 - 100% B over 3.5mins; hold at 100% B for 1.50mins; return to 0% B over 0.50mins. Positive and negative electrospray ionization was employed.

### Intermediate 1: (3aS,4S,6R,6aR)-6-[2-Chloro-6-(2,2-diphenyl-ethylamino)-purin-9-yl]-2,2-dimethyl-tetrahydro-furo[3,4-d][1,3]dioxole-4-carboxylic acid hydrazide

(3aS,4S,6R,6aR)-6-[2-Chloro-6-(2,2-diphenyl-ethylamino)-purin-9-yl]-2,2-dimethyl-tetrahydro-furo[3,4-d][1,3]dioxole-4-carboxylic acid [Preparation 4 from WO94/17090] (200mg, 0.4mmol) in dry dimethylformamide (2mL) was treated with HBTU (152mg, 0.4mmol) and di-isopropylethylamine (129mg, 0.18mL, 1mmol) and the reaction stirred at room temperature under nitrogen for 15 minutes. Hydrazine hydrate (20mg, 0.019mmol) was added and the reaction stirred at room temperature for a further 20 hours. The reaction mixture was partitioned between ethyl acetate (100mL) and saturated ammonium chloride solution (100mL). The organic phase was washed with a further portion of saturated ammonium chloride solution, 2N citric acid (2 x 100mL), saturated sodium bicarbonate (2 x 100mL), dried (MgSO₄) and concentrated *in vacuo* to afford the titled compound as pale coloured foam (0.158g). LC-MS System A Rt.=. 4.73 min., *m*/*z* 550 (MH⁺)

### Intermediate 2: {2-Chloro-9-[6R-(5-(4H-[1,2,4]triazol-3-yl))-2,2-dimethyl-tetrahydro-(3aR,6aR)-furo[3,4-d][1,3]dioxol-4R-yl]-9H-purin-6-yl}-(2,2-diphenyl-ethyl)-amine

Intermediate 1 (2.500g), ethyl formimidate hydrochloride (0.748g, 6.8mmol) and triethylamine (25.8ml) in ethanol (20ml) were heated to reflux for 68 hrs.. Solvent was removed *in vacuo* and the residue was purified by column chromatography on flash silica twice firstly with ethylacetate-cyclohexane (1:1 to neat ethyl acetate), then with cyclohexane-ethyl acetate (10:1, 5:1, 2:1, 1:1, 1:2 and then neat ethyl acetate) to give the title compound as a crisp orange foam (0.185g). TLC SiO₂ (neat ethylacetate, visualised by UV light) Rf = 0.27

### Intermediate 3: (2R,3R,4S,5R)-2-[2-chloro-6-(2,2-diphenyl-ethylamino)-purin-9-yl]-5-(4H-[1,2,4]triazol-3-yl)-tetrahydro-furan-3,4-diol acetate

Intermediate 2 (0.450g, 0.82mmol), acetic acid (20ml) and water (5ml) were heated at 100° C for 3 hrs.. Upon cooling the solvent was removed *in vacuo* and azeotroped with ethyl acetate (50ml). Purification using column chromatography on flash silica eluted with dichloromethane, ethanol and 880 ammonia (100:10:1) furnished the title compound as a cream coloured solid (0.317g) TLC SiO₂ (dichloromethane, methanol and 880 ammonia (100:8:1), visualised by UV light) Rf = 0.14

### Example 1: (2R,3R,4S,5R)-2-[2-(trans-4-Amino-cyclohexylamino)-6-(2,2-diphenyl-ethylamino)-purin-9-yl]-5-(4H-[1,2,4]triazol-3-yl)-tetrahydro-furan-3,4-diol formate

Intermediate 3 (0.026g, 0.05mmol) and trans-1,4-diaminocyclohexane (0.029g, 0.25mmol) in anhydrous DMSO (1.2ml) in a sealed vial (e.g. Reacti-vial™) were heated at 100° C for 71 hrs.. The reaction mixture was diluted with acetonitrile and water (2ml, 1:1) containing 0.1% formic acid. and purified with using Autoprep. HPLC to afford the title compound after freeze drying as a brown coloured solid (0.010g). LC/MS system A Rₜ= 3.51 mins, *m*/*z* = 597 MH⁺

### Example 2: (2R,3R,4S,5R)-2-{6-(2,2-Diphenyl-ethylamino)-2-[2-(1-methyl-1H-imidazol-4-yl)-ethylamino]-purin-9-yl}-5-(4H-[1,2,4]triazol-3-yl)-tetrahydro-furan-3,4-diol formate

Example 2 was prepared in an analogous manner to Example 1 with 1-methylhistamine (0.031g, 0.25mmol) heating for 69 hrs. to afford the title compound after freeze drying as a cream coloured solid (0.012g). LC/MS system A Rₜ = 3.56 mins, *m*/*z* = 608 MH⁺

### Example 3: (2R,3R,4S,5R)-2-[6-(2,2-Diphenyl-ethylamino)-2-(pyrrolidin-3R-ylamino)-purin-9-yl]-5-(4H-[1,2,4]triazol-3-yl)-tetrahydro-furan-3,4-diol formate

Example 3 was prepared in an analogous manner to Example 1 with (3R)-(+)-3-aminopyrrolidine (0.027g, 0.25mmol) to afford the title compound after freeze drying as a cream coloured solid (0.010g).
LC/MS system A Rₜ= 3.55 mins, *m*/*z* = 569 MH⁺

### Example 4: (2R,3R,4S,5R)-2-[6-(2,2-Diphenyl-ethylamino)-2-(1S-hydroxymethyl-2-methyl-propylamino)-purin-9-yl]-5-(4H-[1,2,4]triazol-3-yl)-tetrahydro-furan-3,4-diol formate

Example 4 was prepared in an analogous manner to Example 1 with L-2-amino-3-methylbutanol (0.026g, 0.25mmol) but heated for a further 69 hrs.. Further valinol (0.026g, 0.25mmol) was added and heated at 100° C for 20 hrs. to afford the title compound after freeze drying as a cream coloured solid (0.007g). LC/MS system A Rₜ= 4.06 mins, *m*/*z* = 586 MH⁺

### Example 5: (2R,3R,4S,5R)-2-[6-(2,2-Diphenyl-ethylamino)-2-(2-pyridin-2-yl-ethylamino)-purin-9-yl]-5-(4H-[1,2,4]triazol-3-yl)-tetrahydro-furan-3,4-diol formate

Example 5 was prepared in an analogous manner to Example 1 with 2-(2-amino ethyl)pyridine (0.031g, 0.25mmol) to afford the title compound after freeze drying as a cream coloured solid (0.011g). LC/MS system A Rₜ= 3.73 mins, *m*/*z* = 605 MH⁺

### Example 6: (2R,3R,4S,5R)-2-[2-Cyclopentylamino-6-(2,2-diphenyl-ethylamino)-purin-9-yl]-5-(4H-[1,2,4]triazol-3-yl)-tetrahydro-furan-3,4-diol formate

Example 6 was prepared in an analogous manner to Example 1 with cyclopentylamine (0.021g, 0.25mmol) followed by a second addition of cyclopentylamine (0.021g, 0.25mmol) heated for a further 69 hrs. to afford the title compound after freeze drying as a cream coloured solid (0.003g). LC/MS system B Rₜ= 3.21 mins, *m*/*z =* 568 MH⁺

### Example 7: N-{2-[9-[3R,4S-Dihydroxy-5R-(4H-[1,2,4]triazol-3-yl)-tetrahydro-furan-2R-yl]-6-(2,2-diphenyl-ethylamino)-9H-purin-2-ylamino]-ethyl}-guanidine formate

Example 7 was prepared in an analogous manner to Example 1 with ethylenediamine (0.015g, 0.25mmol). To the reaction mixture was added 1H-pyrazole-1-carboxamidine monohydrochloride (0.073g, 0.5mmol) and imidazole (0.034g, 0.5mmol) and heated at 50°C for 24 hrs.. The reaction mixture was diluted with acetonitrile and water (2ml, 1:1) containing 0.1% formic acid. and purified with using Autoprep. HPLC to afford the title compound after freeze drying as a cream coloured solid (0.008g). LC/MS system B Rₜ = 2.41 mins, *m*/*z* = 585 MH⁺

### Example 8: (2R,3R,4S,5R)-2-[6-(2,2-Diphenyl-ethylamino)-2-(1S-hydroxymethyl-2-phenyl-ethylamino)-purin-9-yl]-5-(4H-[1,2,4]triazol-3-yl)-tetrahydro-furan-3,4-diol formate

Example 8 was prepared in an analogous manner to Example 1 with 3-(S)-(-)2-amino-3-phenyl propanol (0.038g, 0.25mmol) to afford the title compound after freeze drying as a cream coloured solid (0.010g). LC/MS system A Rₜ = 4.20 mins, *m*/*z* = 634 MH⁺

### Example 9: (2R,3R,4S,5R)-2-[6-(2,2-Diphenyl-ethylamino)-2-(2-piperidin-1-yl-ethylamino)-purin-9-yl]-5-(4H-[1,2,4]triazol-3-yl)-tetrahydro-furan-3,4-diol formate

Example 9 was prepared in an analogous manner to Example 1 2-piperidinoethylamine (0.032g, 0.25mmol) to afford the title compound after freeze drying as a cream coloured solid (0.013g).
LC/MS system A Rₜ= 3.59 mins, *m*/*z* = 611 MH⁺

The compounds of the Examples were tested in screen (1) (agonist activity against receptor sub-types) and the results obtained were as follows:

| Example No. | A2a | A3 | A1 |
|---|---|---|---|
| 1 | 11.37 | > 353 | 1098 |
| 2 | 1.08 | > 430 | 1003.4 |
| 3 | 10.49 | > 306 | 586.92 |
| 4 | 3.79 | > 306 | 670.92 |
| 5 | 19.3 | > 397 | 3329 |
| 6 | 16.96 | > 305 | 1627 |
| 7 | 19.01 | > 368 | 2577 |
| 8 | 26.74 | > 326 | 280.97 |
| 9 | 8.13 | > 472 | 969.8 |
| Values given in the Table are EC₅₀ values as a ratio of that of NECA. | | | |

### ABBREVIATIONS

- TMS: trimethylsilyl
- TFA: trifluoroacetic acid
- DMF: N,N-dimethylformamide
- NECA: N-ethylcarboxamideadenosine
- DMAP: 4-dimethylaminopyridine
- TEMPO: 2,2,6,6-tetramethyl-1-piperidinyloxy, free radical
- TMSOTf: Trimethylsilyltrifluoromethylsulphonate
- DBU: 1,8-diazabicyclo[5.4.0]undec-7-ene
- BSA: bistrimethylsilylacetamide
- DCM: dichloromethane
- DAST: diethylaminosulphur trifluoride
- Ph: phenyl
- CDI: carbonyldiimidazole
- EEDQ: 2-ethoxy-1-ethoxycarbonyl-1,2 dihydroquinone
- NSAID: non-steroidal antiinflammatory drug
- HBTU: 2-(IH-Benzotriazole-1-yl)-1,1,3,3-tetramethyluronium hexafluorophosphate
- DMSO: dimethylsulphoxide
- DEAD: diethylazocarboxylate

## Claims

1. A compound of formula (I): 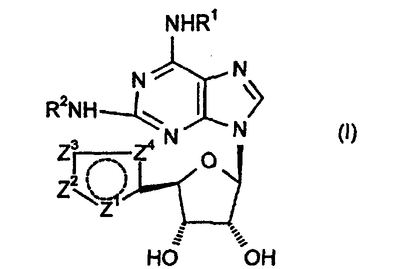 wherein R¹ and R² independently represent a group selected from:
(i) C₃₋₈cycloalkyl-;
(ii) hydrogen;
(iii) aryl₂CHCH₂-;
(iv) C₃₋₈cycloalkylC₁₋₆alkyl-;
(v) C₁₋₈alkyl-;
(vi) arylC₁₋₆alkyl-;
(vii) R⁴R⁵N-C₁₋₆alkyl-;
(viii) C₁₋₆alkyl-CH(CH₂OH)-;
(ix) arylC₁₋₅alkyl-CH(CH₂OH)-;
(x) arylC₁₋₅alkyl-C(CH₂OH)₂-;
(xi) C₃₋₈cycloalkyl independently substituted by one or more (e.g. 1, 2 or 3) -(CH₂)ₚR⁶ groups;
(xii) H₂NC(=NH)NHC₁₋₆alkyl-;
(xiii) a group of formula 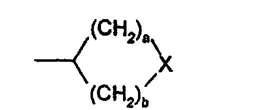 or such a group in which one methylene carbon atom adjacent to X, or both if such exist, is substituted by methyl;
(xiv) -C₁₋₆alkyl-OH;
(xv) -C₁₋₈haloalkyl;
(xvi) a group of formula 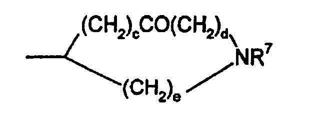
(xvii) aryl; and
(xviii) -(CH₂)_{f}SO₂NH_{g}(C₁₋₄alkyl-)_{2-g} or -(CH₂)_{f}SO₂NH_{g}(arylC₁₋₄alkyl-)_{2-g} where f is 2 or 3 and g is an integer 0 to 2;
Z¹, Z², Z³ and Z⁴ together with the carbon atom form a 5-membered heterocyclic aromatic ring;
R⁴ and R⁵ independently represent hydrogen, C₁₋₆alkyl, aryl, arylC₁₋₆alkyl- or NR⁴R⁵ together may represent pyridinyl, pyrrolidinyl, piperidinyl, morpholinyl, azetidinyl, azepinyl, piperazinyl or N-C₁₋₆alkylpiperazinyl;
R⁶ represents OH, NH₂, NHCOCH₃ or halogen;
R⁷ represents hydrogen, C₁₋₆alkyl, -C₁₋₆alkylaryl or -COC₁₋₆alkyl;
X represents NR⁷, O, S, SO or SO₂;
p represents 0 or 1;
a and b independently represent an integer 0 to 4 provided that a + b is in the range 3 to 5;
c, d and e independently represent an integer 0 to 3 provided that c + d + e is in the range 2 to 3;
and salts and solvates thereof.

2. A compound according to claim 1 wherein R¹ and R² do not both represent hydrogen.

3. A compound according to claim 1 or claim 2 wherein R¹ represents aryl₂CHCH₂-, C₁₋₈alkyl-, hydrogen or arylC₁₋₆alkyl.

4. A compound according to any one of claims 1 to 3 wherein R¹ represents Ph₂CHCH₂.

5. A compound according to any one of claims 1 to 4 wherein R² represents ethyl-piperidin-1-yl, PhCH₂CH(CH₂OH)-, -CH(CH₂OH)CH(CH₃)₂, trans-4-amino-cyclohexyl, 2-(1-methyl-1H-imidazoyl-4-yl)CH₂CH₂-, pyrrolidin-3-yl, ethyl-pyridin-2-yl, H₂NC(=NH)NH(CH₂)₂-, or cyclopentyl.

6. A compound according to any one of claims 1 to 5 wherein R² represents -CH(CH₂OH)CH(CH₃)₂ or 2-(1-methyl-1H-imidazoyl-4-yl)CH₂CH₂-.

7. A compound according to any one of claims 1 to 6 wherein R⁴ and R⁵ independently represent hydrogen or aryl or NR⁴R⁵ together may represent pyrrolidinyl, piperidinyl, morpholinyl, azetidinyl, azepinyl, piperazinyl or N-methylpiperazinyl.

8. A compound according to any one of claims 1 to 7 wherein R⁶ represents OH or NH₂.

9. A compound according to any one of claims 1 to 8 wherein X represents NR⁷, O, S or SO₂.

10. A compound according to any one of claims 1 to 9 wherein the moiety 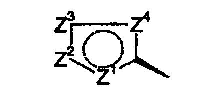 represents one of the following groups:

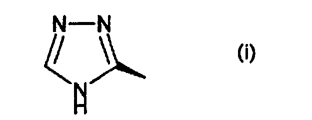

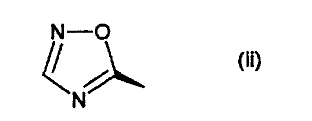

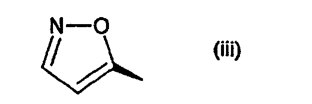

11. A compound according to any one of claims 1 to 10 wherein the moiety 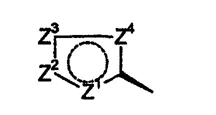 represents

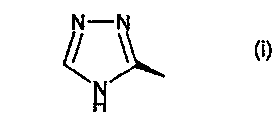

12. A compound of formula (I) which is
(2R,3R,4S,5R)-2-[2-(trans-4-Amino-cyclohexylamino)-6-(2,2-diphenyl-ethylamino)-purin-9-yl]-5-(4H-[1,2,4]triazol-3-yl)-tetrahydro-furan-3,4-diol;
(2R,3R,4S,5R)-2-{6-(2,2-Diphenyl-ethylamino)-2-[2-(1-methyl-1H-imidazol-4-yl)-ethylamino]-purin-9-yl}-5-(4H-[1,2,4]triazol-3-yl)-tetrahydro-furan-3,4-diol;
(2R,3R,4S,5R)-2-[6-(2,2-Diphenyl-ethylamino)-2-(pyrrolidin-3R-ylamino)-purin-9-yl]-5-(4H-[1,2,4]triazol-3-yl)-tetrahydro-furan-3,4-diol;
(2R,3R,4S,5R)-2-[6-(2,2-Diphenyl-ethylamino)-2-(1S-hydroxymethyl-2-methyl-propylamino)-purin-9-yl]-5-(4H-[1,2,4]triazol-3-yl)-tetrahydro-furan-3,4-diol;
(2R,3R,4S,5R)-2-[6-(2,2-Diphenyl-ethylamino)-2-(2-pyridin-2-yl-ethylamino)-purin-9-yl]-5-(4H-[1,2,4]triazol-3-yl)-tetrahydro-furan-3,4-diol;
(2R,3R,4S,5R)-2-[2-Cyclopentylamino-6-(2,2-diphenyl-ethylamino)-purin-9-yl]-5-(4H-[1,2,4]triazol-3-yl)-tetrahydro-furan-3,4-diol;
N-{2-[9-[3R,4S-Dihydroxy-5R-(4H-[1,2,4]triazol-3-yl)-tetrahydro-furan-2R-yl]-6-(2,2-diphenyl-ethylamino)-9H-purin-2-ylamino]-ethyl}-guanidine;
(2R,3R,4S,5R)-2-[6-(2,2-Diphenyl-ethylamino)-2-(1S-hydroxymethyl-2-phenyl-ethylamino)-purin-9-yl]-5-(4H-[1,2,4]triazol-3-yl)-tetrahydro-furan-3,4-diol;
(2R,3R,4S,5R)-2-[6-(2,2-Diphenyl-ethylamino)-2-(2-piperidin-1-yl-ethylamino)-purin-9-yl]-5-(4H-[1,2,4]triazol-3-yl)-tetrahydro-furan-3,4-diol;
or a salt or solvate of any one thereof.

13. A pharmaceutical composition comprising a compound of formula (I) as defined in any one of claims 1 to 12 or a pharmaceutically acceptable salt or solvate thereof in admixture with one or more pharmaceutically acceptable diluents or carriers.

14. A compound of formula (I) as defined in any one of claims 1 to 12 or a pharmaceutically acceptable salt or solvate thereof for use as a pharmaceutical.

15. Use of a compound of formula (I) as defined in any one of claims 1 to 12 or a pharmaceutically acceptable salt or solvate thereof in the manufacture of a medicament for the treatment of inflammatory diseases.

16. A process for preparation of compounds of formula (I) as defined in any one of claims 1 to 12 which comprises reacting a compound of formula (II) 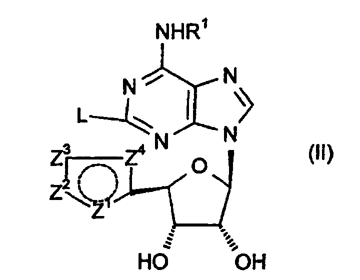 wherein L represents a leaving group or a protected derivative thereof with a compound of formula R²NH₂ or a protected derivative thereof, wherein R¹, R², Z¹, Z², Z³ and Z⁴ are as defined in any one of claims 1 to 12.

17. A process for preparation of compounds of formula (I) where the moiety: 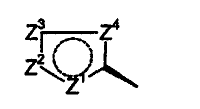 is represented by: 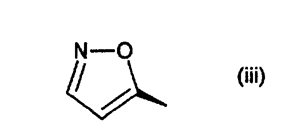 which comprises dehalogenating a compound of formula (VII): 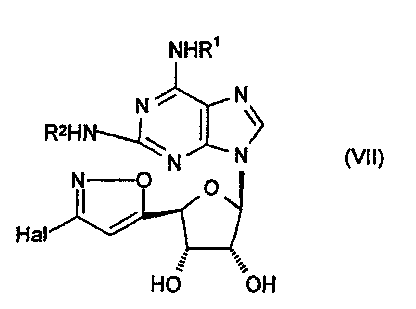 wherein R¹ and R² are as defined in any one of claims 1 to 12 and Hal represents halogen.

18. A compound of formula (II) 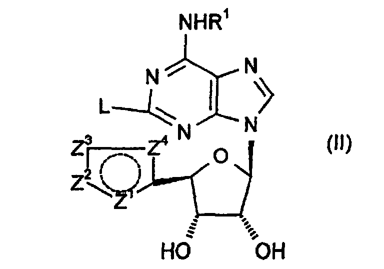 wherein L represents a leaving group or a protected derivative thereof, wherein R¹, R², Z¹, Z², Z³ and Z⁴ are as defined in any one of claims 1 to 12, with the proviso that said compound is not a compound of formula: 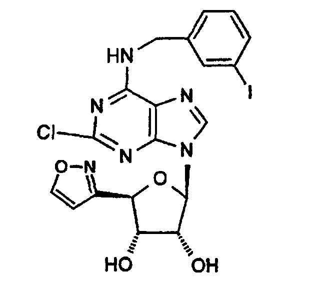 or a compound of formula:

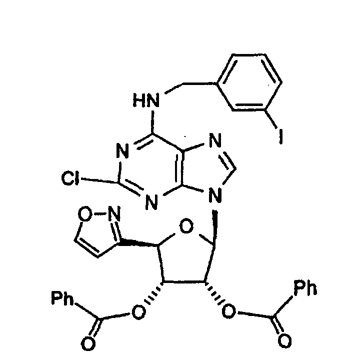

19. A compound of formula (III) 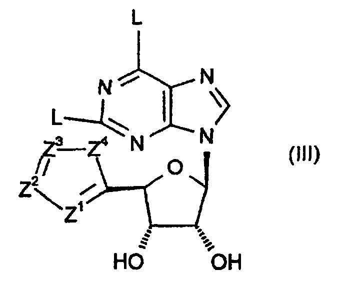 or a protected derivative thereof, wherein L represents a leaving group or a protected derivative thereof and Z¹, Z², Z³ and Z⁴ are as defined in any one of claims 1 to 12, with the proviso that said compound is not a compound of formula:

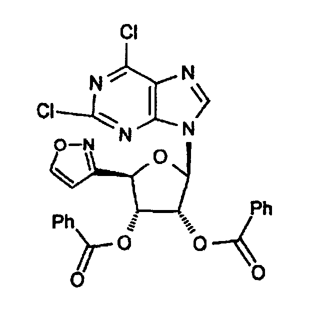

20. A compound of formula (IV) 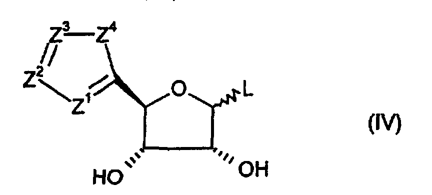 wherein L represents a leaving group, or a protected derivative thereof and Z¹, Z², Z³ and Z⁴ are as defined in any one of claims 1 to 12, with the proviso that said compound is not a compound of formula: 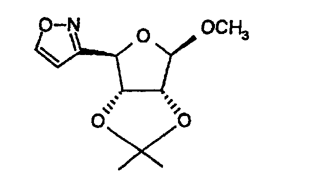 or a compound of formula: 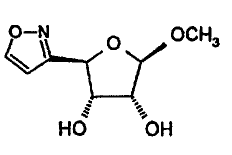 or a compound of formula :

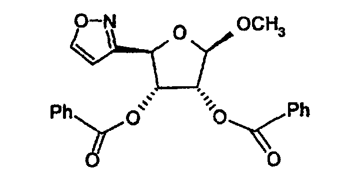

21. A compound of formula (V) 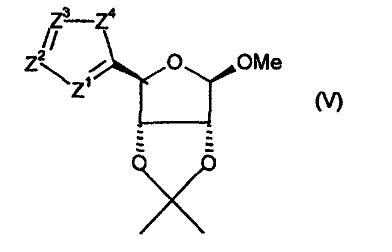 wherein Z¹, Z², Z³ and Z⁴ are as defined in any one of claims 1 to 12, with the proviso that said compound is not a compound of formula:

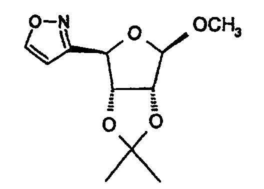

22. A compound of formula (VI) 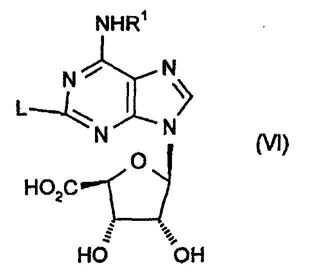 wherein L represents a leaving group or a protected derivative thereof and R¹ is as defined in any one of claims 1 to 12, with the proviso that said compound is not a compound of formula: 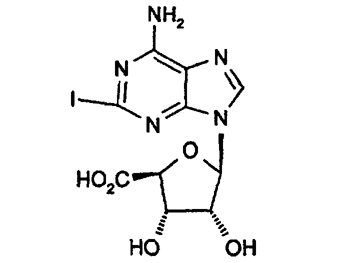 and with the proviso that when R¹ represents Ph₂CHCH₂, L does not represent chlorine.

## Patentansprüche

1. Verbindung der Formel (I): 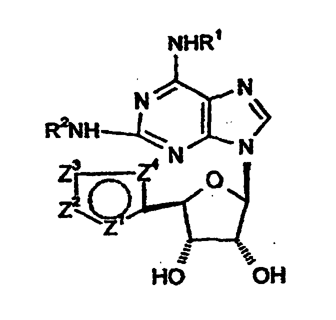 worin R¹ und R² unabhängig eine Gruppe darstellen, ausgewählt aus:
(i) C₃₋₈-Cycloalkyl-;
(ii) Wasserstoff;
(iii) Aryl₂CHCH₂-;
(iv) C₃₋₈-Cycloalkyl-C₁₋₆-alkyl-;
(v) C₁₋₈-Alkyl-;
(vi) Aryl-C₁₋₆-alkyl-;
(vii) R⁴R⁵N-C₁₋₆-Alkyl-;
(viii) C₁₋₆-Alkyl-CH(CH₂OH)-;
(ix) Aryl-C₁₋₅-alkyl-CH(CH₂OH)-;
(x) Aryl-C₁₋₅-alkyl-C(CH₂OH)₂-;
(xi) C₃₋₈-Cycloalkyl, das unabhängig mit einer oder mehreren (z.B. 1, 2 oder 3) -(CH₂)ₚR⁶-Gruppen substituiert ist;
(xii) H₂NC(=NH)NHC₁₋₆-Alkyl-;
(xiii) einer Gruppe der Formel 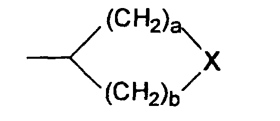 oder einer solchen Gruppe, in der ein zu X benachbartes Methylen-Kohlenstoffatom oder beide, falls solche existieren, mit Methyl substituiert ist;
(xiv) -C₁₋₆-Alkyl-OH;
(xv) -C₁₋₈-Halogenalkyl;
(xvi) einer Gruppe der Formel 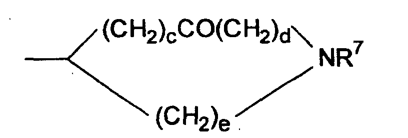
(xvii) Aryl; und
(xviii) -(CH₂)_{f}SO₂NH_{g}(C₁₋₄-Alkyl-)_{2-g} oder -(CH₂)_{f}SO₂NH_{g}(Aryl-C₁₋₄-alkyl-)_{2-g}, worin f 2 oder 3 ist und g eine ganze Zahl von 0 bis 2 ist;
Z¹, Z², Z³ und Z⁴ zusammen mit dem Kohlenstoffatom einen 5-gliedrigen heterocyclischen aromatischen Ring bilden;
R⁴ und R⁵ unabhängig Wasserstoff, C₁₋₆-Alkyl, Aryl oder Aryl-C₁₋₆-alkyl- darstellen oder NR⁴R⁵ zusammen Pyridinyl, Pyrrolidinyl, Piperidinyl, Morpholinyl, Azetidinyl, Azepinyl, Piperazinyl oder N-C₁₋₆-Alkylpiperazinyl darstellen kann;
R⁶ OH, NH₂, NHCOCH₃ oder Halogen darstellt;
R₇ Wasserstoff, C₁₋₆-Alkyl, -C₁₋₆-Alkylaryl oder -COC₁₋₆-Alkyl darstellt;
X NR⁷, O, S, SO oder SO₂ darstellt;
p 0 oder 1 darstellt;
a und b unabhängig eine ganze Zahl von 0 bis 4 darstellen, vorausgesetzt, dass a + b im Bereich von 3 bis 5 ist;
c, d und e unabhängig eine ganze Zahl von 0 bis 3 darstellen, vorausgesetzt, dass c + d + e im Bereich von 2 bis 3 ist; und Salze und Solvate davon.

2. Verbindung gemäss Anspruch 1, worin R¹ und R² nicht beide Wasserstoff darstellen.

3. Verbindung gemäss Anspruch 1 oder 2, worin R¹ Aryl₂CHCH₂-, C₁₋₈-Alkyl-, Wasserstoff oder Aryl-C₁₋₆-alkyl darstellt.

4. Verbindung gemäss einem der Ansprüche 1 bis 3, worin R¹ Ph₂CHCH₂- darstellt.

5. Verbindung gemäss einem der Ansprüche 1 bis 4, worin R² Ethyl-piperidin-1-yl, PhCH₂CH(CH₂OH]-, -CH(CH₂OH)CH(CH₃)₂, trans-4-Amino-cyclohexyl, 2-(1-Methyl-lH-imidazolyl-4-yl)CH₂CH₂-, Pyrrolidin-3-yl, Ethyl-pyridin-2-yl, H₂NC(=NH)NH(CH₂)₂- oder Cyclopentyl darstellt.

6. Verbindung gemäss einem der Ansprüche 1 bis 5, worin R² -CH(CH₂OH)CH(CH₃)₂ oder 2-(1-Methyl-1H-imidazolyl)CH₂CH₂darstellt.

7. Verbindung gemäss einem der Ansprüche 1 bis 6, worin R⁴ und R⁵ unabhängig Wasserstoff oder Aryl darstellen oder NR⁴R⁵ zusammen Pyrrolidinyl, Piperidinyl, Morpholinyl, Azetidinyl, Azepinyl, Piperazinyl oder N-Methylpiperazinyl darstellen kann.

8. Verbindung gemäss einem der Ansprüche 1 bis 7, worin R⁶ OH oder NH₂ darstellt.

9. Verbindung gemäss einem der Ansprüche 1 bis 8, worin X NR⁷, O, S oder SO₂ darstellt.

10. Verbindung gemäss einem der Ansprüche 1 bis 9, worin die Einheit 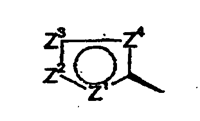 eine der folgenden Gruppen darstellt

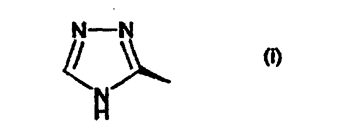

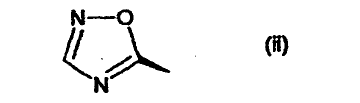

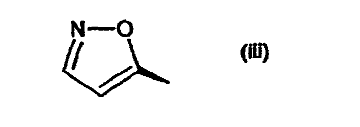

11. Verbindung gemäss einem der Ansprüche 1 bis 10, worin die Einheit 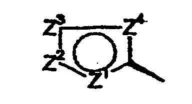 durch 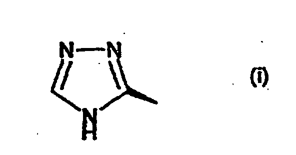 dargestellt wird.

12. Verbindung der Formel (I), die:
(2R,3R,4S,5R)-2-[2-(trans-4-Amino-cyclohexylamino)-6-(2,2-diphenyl-ethylamino)-purin-9-yl]-5-(4H-[1,2,4]triazol-3-yl) -tetrahydro-furan-3,4-diol;
(2R,3R,4S,5R)-2-{6-(2,2-Diphenyl-ethylamino)-2-[2-(1-methyl-1H-imidazol-4-yl)-ethylamino]-purin-9-yl}-5-(4H-[1,2,4]triazol-3-yl)-tetrahydro-furan-3,4-diol;
(2R,3R,4S,5R)-2-[6-(2,2-Diphenyl-ethylamino)-2-(pyrrolidin-3R-ylamino)-purin-9-yl]-5-(4H-[1,2,4]triazol-3-yl)-tetrahydro-furan-3,4-diol;
(2R,3R,4S,5R)-2-{6-(2,2-Diphenyl-ethylamino)-2-(1Shydroxymethyl-2-methyl-propylamino)-purin-9-yl}-5-(4H-[1,2,4]triazol-3-yl)-tetrahydro-furan-3,4-diol;
(2R,3R,4S,5R)-2-[6-(2,2-Diphenyl-ethylamino)-2-(pyridin-2-yl-ethylamino)-purin-9-yl]-5-(4H-[1,2,4]triazol-3-yl)-tetrahydro-furan-3,4-diol;
(2R,3R,4S,5R)-2-[6-(2-Cyclopentylamino)-6-(2,2-diphenylethylamino)-purin-9-yl]-5-(4H-[1,2,4]triazol-3-yl)-tetrahydro-furan-3,4-diol;
N-{2-[9-[3R,4S-Dihydroxy-5R-(4H-[1,2,4]triazol-3-yl)-tetrahydro-furan-2R-yl]-6-(2,2-diphenyl-ethylamino)-9H-purin-2-ylamino]-ethyl}-guanidin;
(2R,3R,4S,5R)-2-[6-(2,2-Diphenyl-ethylamino)-2-(1Shydroxymethyl-2-phenyl-ethylamino)-purin-9-yl]-5-(4H-[1,2,4]triazol-3-yl)-tetrahydro-furan-3,4-diol;
(2R,3R,4S,5R)-2-[6-(2,2-Diphenyl-ethylamino)-2-(2-piperidin-1-yl-ethylamino)-purin-9-yl]-5-(4H-[1,2,4]triazol-3-yl)-tetrahydro-furan-3,4-diol;
oder ein Salz oder Solvat von jedem davon ist.

13. Pharmazeutische Zusammensetzung, die eine Verbindung der Formel (I) wie in einem der Ansprüche 1 bis 12 definiert oder ein pharmazeutisch akzeptables Salz oder Solvat davon im Gemisch mit einem oder mehreren pharmazeutisch akzeptablen Verdünnungsmitteln oder Trägern umfasst.

14. Verbindung der Formel (I) wie in einem der Ansprüche 1 bis 12 definiert oder ein pharmazeutisch akzeptables Salz oder Solvat davon zur Verwendung als Pharmazeutikum.

15. Verwendung einer Verbindung der Formel (I) wie in einem der Ansprüche 1 bis 12 definiert oder eines pharmazeutisch akzeptablen Salzes oder Solvats davon in der Herstellung eines Medikaments zur Behandlung von entzündlichen Erkrankungen.

16. Verfahren zur Herstellung von Verbindungen der Formel (I) wie in einem der Ansprüche 1 bis 12 definiert, welches das Umsetzen einer Verbindung der Formel (II): 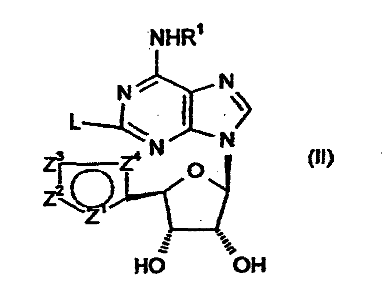 worin L eine Abgangsgruppe darstellt, oder eines geschützten Derivats davon mit einer Verbindung der Formel R²NH₂ oder einem geschützten Derivat davon umfasst, worin R¹, R², Z¹, Z², Z³ und Z⁴ wie in einem der Ansprüche 1 bis 12 definiert sind.

17. Verfahren zur Herstellung von Verbindungen der Formel (I), worin die Einheit: 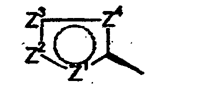 durch: 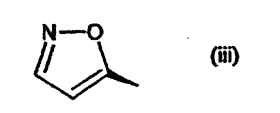 dargestellt wird, welches das Dehalogenieren einer Verbindung der Formel (VII): 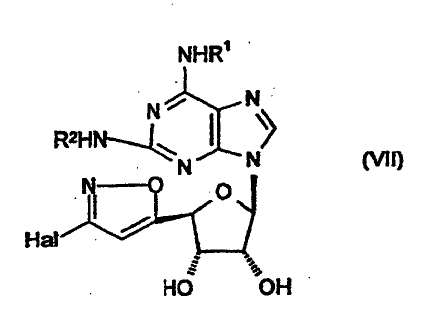 worin R¹ und R² wie in einem der Ansprüche 1 bis 12 definiert sind und Hal Halogen darstellt, umfasst.

18. Verbindung der Formel (II) 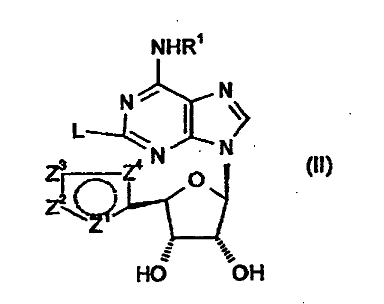 worin L eine Abgangsgruppe darstellt oder ein geschütztes Derivat davon, worin R¹, R², Z¹, Z², Z³ und Z⁴ wie in einem der Ansprüche 1 bis 12 definiert sind, mit der Maßgabe, dass die Verbindung nicht eine Verbindung der Formel: 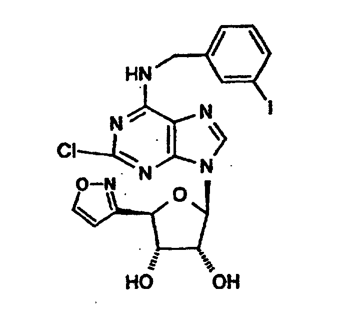 oder eine Verbindung der Formel 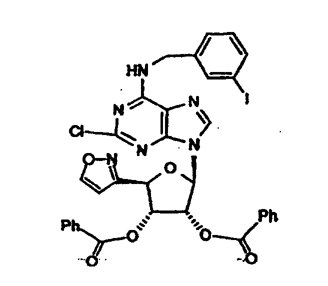 ist.

19. Verbindung der Formel (III) 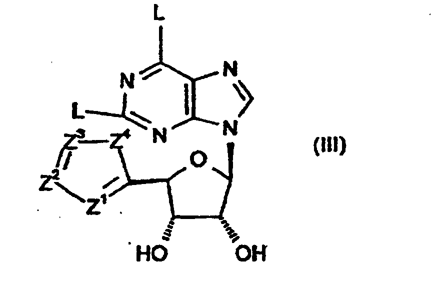 oder ein geschütztes Derivat davon, worin L eine Abgangsgruppe oder ein geschütztes Derivat davon ist und Z¹, Z², Z³ und Z⁴ wie in einem der Ansprüche 1 bis 12 definiert sind, mit der Maßgabe, dass die Verbindung nicht eine Verbindung der Formel: 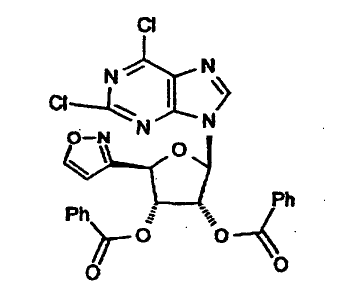 ist.

20. Verbindung der Formel (IV) 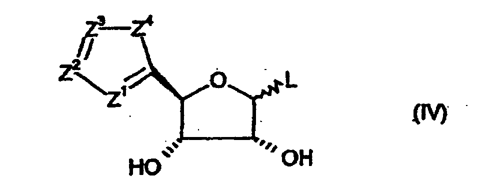 worin L eine Abgangsgruppe darstellt oder ein geschütztes Derivat davon und Z¹, Z², Z³ und Z⁴ wie in einem der Ansprüche 1 bis 12 definiert sind, mit der Maßgabe, dass die Verbindung nicht eine Verbindung der Formel 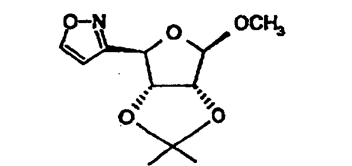 oder eine Verbindung der Formel 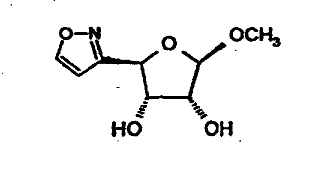 oder eine Verbindung der Formel 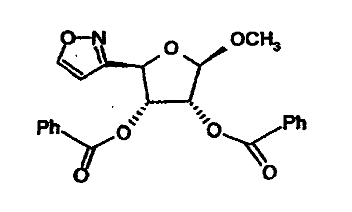 ist.

21. Verbindung der Formel (V) 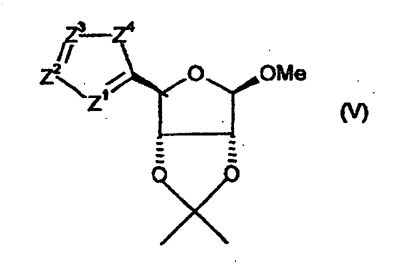 worin Z¹, Z², Z³ und Z⁴ wie in einem der Ansprüche 1 bis 12 definiert sind, mit der Maßgabe, dass die Verbindung nicht eine Verbindung der Formel 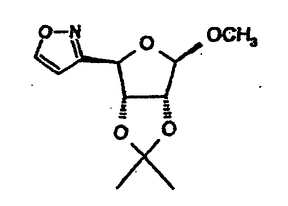 ist.

22. Verbindung der Formel (VI) 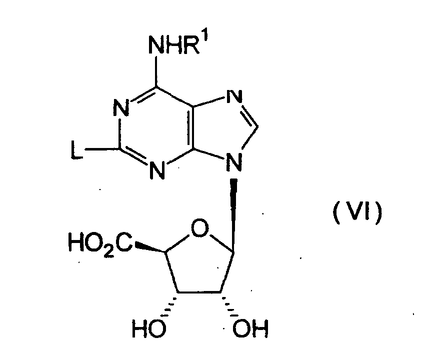 worin L eine Abgangsgruppe darstellt oder ein geschütztes Derivat davon und R¹ wie in einem der Ansprüche 1 bis 12 definiert ist, mit der Massgabe, dass die Verbindung nicht eine Verbindung der Formel: 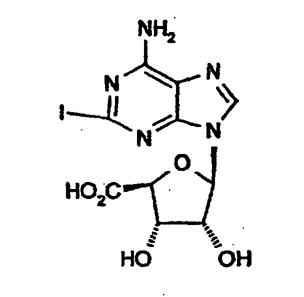 ist, und mit der Maßgabe, dass L nicht Chlor darstellt, wenn R¹ Ph₂CHCH₂ darstellt.

## Revendications

1. Composé de formule (I): 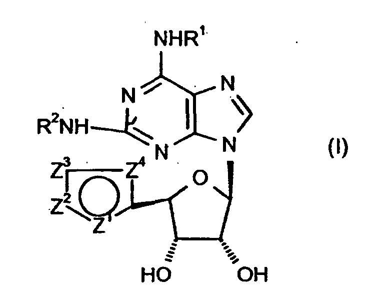 dans laquelle R¹ et R² représentent indépendamment un groupe choisi parmi :
(i) un groupe cycloalkyle en C₃₋₈ ;
(ii) un atome d'hydrogène ;
(iii) un groupe aryl₂CHCH₂-;
(iv) un groupe cycloalkyl(en C₃₋₈)alkyl (en C₁₋₆) ;
(v) un groupe alkyle en C₁₋₈ ;
(vi) un groupe arylalkyle en C₁₋₆ ;
(vii) un groupe R⁴R⁵N-alkyle en C₁₋₆ ;
(viii) un groupe alkyl(en C₁₋₆)-CH(CH₂OH)-;
(ix) un groupe arylalkyl(en C₁₋₅)-CH(CH₂OH)-;
(x) un groupe arylalkyl(en C₁₋₅)-C(CH₂OH)₂-;
(xi) un groupe cycloalkyle en C₃₋₈ indépendamment substitué par un ou plusieurs (par exemple 1, 2 ou 3) groupes -(CH₂)ₚR⁶ ;
(xii) un groupe H₂NC(=NH)NHalkyle en C₁₋₆;
(xiii) un groupe de formule 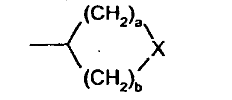 ou un tel groupe dans lequel l'un des atomes de carbone de méthylène adjacent à X, ou les deux si cela est le cas, sont substitués par un groupe méthyle ;
(xiv) un groupe alkyl(en C₁₋₆)-OH ;
(xv) un groupe haloalkyle en C₁₋₈ ;
(xvi) un groupe de formule 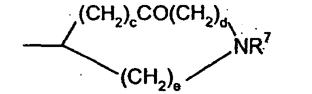
(xvii) un groupe aryle ; et
(xviii) un groupe -(CH₂)_{f}SO₂NH_{g}(alkyle en C₁₋₄)_{2-g} ou -(CH₂)_{f}SO₂NH_{g}(arylalkyle en C₁₋₄)_{2-g} où f est 2 ou 3 et g est un nombre entier de 0 à 2;
Z¹, Z², Z³ et Z⁴ forment avec l'atome de carbone un noyau aromatique hétérocyclique à 5 chaînons ;
R⁴ et R⁵ représentent indépendamment un atome d'hydrogène, un groupe alkyle en C₁₋₆, aryle, arylalkyle en C₁₋₆ ou NR⁴R⁵ peuvent représenter ensemble un groupe pyridinyle, pyrrolidinyle, pipéridinyle, morpholinyle, azétidinyle, azépinyle, pipérazinyle ou N-alkyl(en C₁₋₆)-pipérazinyle ;
R⁶ représente OH, NH₂, NHCOCH₃ ou un atome d'halogène ;
R⁷ représente un atome d'hydrogène, un groupe alkyle en C₁₋₆, un groupe alkyl(en C₁₋₆)-aryle ou -CO-alkyle en C₁₋₆ ;
X représente NR⁷, O, S, SO ou SO₂;
p représente 0 ou 1;
a et b représentent indépendamment un nombre entier de 0 à 4 à condition que a + b soit compris dans la gamme de 3 à 5 ;
c, d et e représentent indépendamment un nombre entier de 0 à 3 à condition que c + d + e soit compris dans la gamme de 2 à 3;
et sels et solvates de celui-ci.

2. Composé selon la revendication 1, dans lequel R¹ et R² ne représentent pas tous les deux un atome d'hydrogène.

3. Composé selon la revendication 1 ou la revendication 2, dans lequel R¹ représente un groupe aryl₂CHCH₂-, un groupe alkyle en C₁₋₆, un atome d'hydrogène ou un groupe arylalkyle en C₁₋₆.

4. Composé selon l'une quelconque des revendications 1 à 3, dans lequel R' représente Ph₂CHCH₂-.

5. Composé selon l'une quelconque des revendications 1 à 4, dans lequel R² représente un groupe éthyl-pipéridine-1-yle, PhCH₂CH(CH₂OH)-, -CH(CH₂OH)CH(CH₃)₂, trans-4-amino-cyclohexyle, 2-(1-méthyl-1H-imidazoyl-4-yl)CH₂CH₂-, pyrrolidin-3-yle, éthyl-pyridine-2-yle, H₂NC(=NH)NH(CH₂)₂- ou cyclopentyle.

6. Composé selon l'une quelconque des revendications 1 à 5, dans lequel R² représente un groupe -CH(CH₂OH)CH(CH₃)₂, ou 2-(1-méthyl-1H-imidazoyl-4-yl)CH₂CH₂-.

7. Composé selon l'une quelconque des revendications 1 à 6, dans lequel R⁴ et R⁵ représentent indépendamment un atome d'hydrogène ou un groupe aryle, ou bien NR⁴R⁵ peuvent représenter ensemble un groupe pyrrolidinyle, pipéridinyle, morpholinyle, azétidinyle, azépinyle, pipérazinyle ou N-méthylpipérazinyle.

8. Composé selon l'une quelconque des revendications 1 à 7, dans lequel R⁶ représente OH ou NH₂.

9. Composé selon l'une quelconque des revendications 1 à 8, dans lequel X représente NR⁷, O, S ou SO₂.

10. Composé selon l'une quelconque des revendications 1 à 9, dans lequel le motif 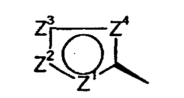 représente l'un des groupes suivants :

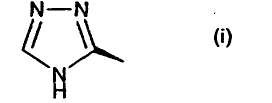

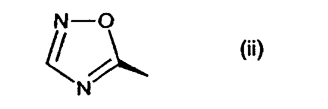

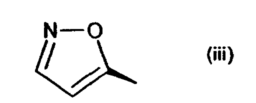

11. Composé selon l'une quelconque des revendications 1 à 10, dans lequel le motif 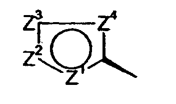 représente le groupe :

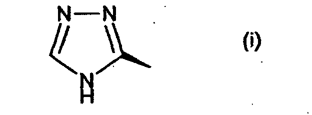

12. Composé de formule (I), qui est
le (2R,3R,4S,5R)-2-[2-(trans-4-amino-cyclohexylamino)-6-(2,2-diphényl-éthylamino)-purin-9-yl]-5-(4H-[1,2,4]triazol-3-yl)-tétrahydro-furan-3,4-diol;
le (2R,3R,4S,5R)-2-{6-(2,2-diphényl-éthylamino)-2-[2-(1-méthyl-1H-imidazol-4-yl)-éthylamino]-purin-9-yl}-5-(4H-[1,2,4]triazol-3-yl)-tétrahydro-furan-3,4-diol;
le (2R,3R,4S,5R)-2-[6-(2,2-diphényl-éthylamino)-2-(pyrrolidin-3R-ylamino)-purin-9-yl]-5-(4H-[1,2,4]triazol-3-yl)-tétrahydro-furan-3,4-diol;
le (2R,3R,4S,5R)-2-[6-(2,2-diphényl-éthylamino)-2-(1S-hydroxyméthyl-2-méthyl-propylamino)-purin-9-yl]-5-(4H-[1,2,4]triazol-3-yl)-tétrahydro-furan-3,4-diol;
le (2R,3R,4S,5R)-2-[6-(2,2-diphényl-éthylamino)-2-(2-pyridin-2-yl-éthylamino)-purin-9-yl]-5-(4H-[1,2,4]triazol-3-yl)-tétrahydrofuran-3,4-diol;
le (2R,3R,4S,5R)-2-[2-cyclopentylamino-6-(2,2-diphényl-éthylamino)-purin-9-yl]-5-(4H-[1,2,4]triazol-3-yl)-tétrahydrofuran-3,4-diol;
la N-{2-[9-[3R,4S-dihydroxy-5R-(4H-[1,2,4]triazol-3-yl)-tétrahydro-furan-2R-yl]-6-(2,2-diphényl-éthylamino)-9H-purin-2-ylamino]-éthyl}-guanidine;
le (2R,3R,4S,5R)-2-[6-(2,2-diphényl-éthylamino)-2-(1S-hydroxyméthyl-2-phényl-éthylamino)-purin-9-yl]-5-(4H-[1,2,4]triazol-3-yl)-tétrahydro-furan-3,4-diol;
le (2R,3R,4S,5R)-2-[6-(2,2-diphényl-éthylamino)-2-(2-pipéridin-1-yl-éthylamino)-purin-9-yl]-5-(4H-[1,2,4]triazol-3-yl)-tétrahydrofuran-3,4-diol;
ou sel ou solvate de l'un quelconque d'entre eux.

13. Composition pharmaceutique comprenant un composé de formule (I) selon l'une quelconque des revendications 1 à 12, ou un sel ou un solvate de celui-ci acceptable du point de vue pharmaceutique en mélange avec un ou plusieurs supports ou diluants acceptables du point de vue pharmaceutique.

14. Composé de formule (I) selon l'une quelconque des revendications 1 à 12, ou sel ou solvate de celui-ci acceptables du point de vue pharmaceutique utile en tant que produit pharmaceutique.

15. Utilisation d'un composé de formule (I) selon l'une quelconque des revendications 1 à 12, ou d'un sel ou d'un solvate de celui-ci acceptable du point de vue pharmaceutique dans la fabrication d'un médicament pour le traitement de maladies inflammatoires.

16. Procédé de préparation de composés de formule (I) selon l'une quelconque des revendications 1 à 12, qui comprend la réaction d'un composé de formule (II) 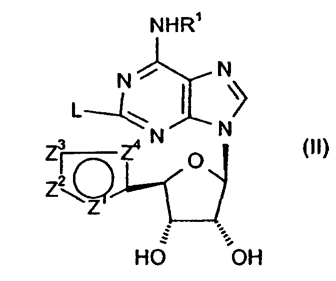 dans laquelle L représente un groupe mobile, ou d'un dérivé protégé de celui-ci, avec un composé de formule R²NH₂ ou un dérivé protégé de celui-ci, dans lequel R¹, R², Z¹, Z², Z³ et Z⁴ sont tels que définis dans l'une quelconque des revendications 1 à 12.

17. Procédé de préparation de composés de formule (I) où le motif 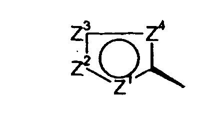 est représenté par : 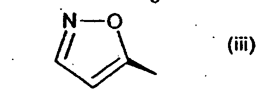 qui comprend la déshalogénation d'un composé de formule (VII): 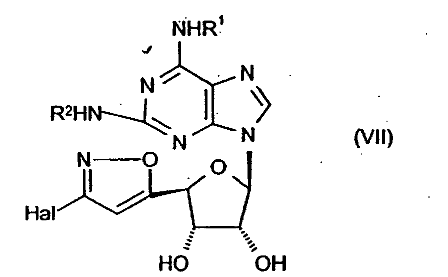 dans laquelle R¹ et R² sont tels que définis selon l'une quelconque des revendications 1 to 12 et Hal représente un atome d'halogène.

18. Composé de formule (II) : 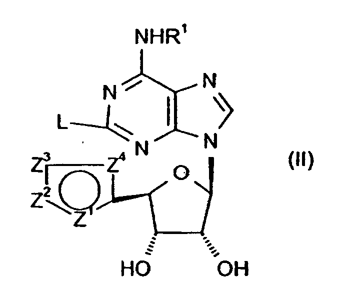 dans laquelle L représente un groupe mobile ou un dérivé protégé de celui-ci et où R¹, R², Z¹, Z², Z³ et Z⁴ sont tels que définis dans l'une quelconque des revendications 1 à 12, à la condition que ce composé ne soit pas un composé de formule 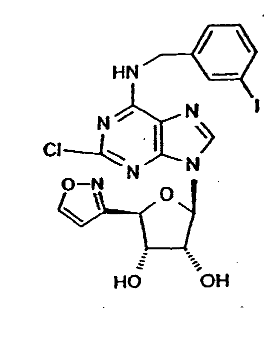 ou un composé de formule :

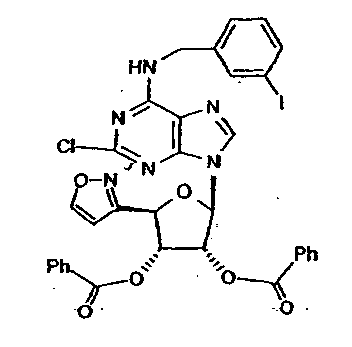

19. Composé de formule (III) : 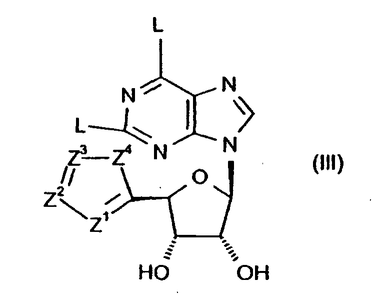 ou dérivé protégé de celui-ci, où L représente un groupe mobile ou un dérivé protégé de celui-ci et Z¹, Z², Z³ et Z⁴ sont tels que définis dans l'une quelconque des revendications 1 à 12, à la condition que ce composé ne soit pas un composé de formule

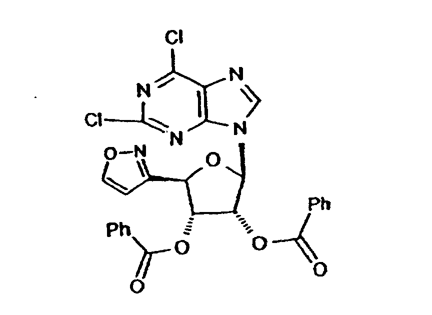

20. Composé de formule (IV): 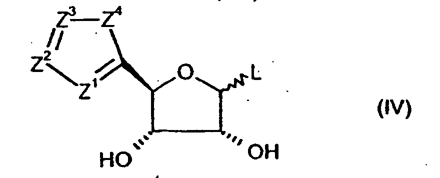 dans laquelle L représente un groupe mobile ou un dérivé protégé de celui-ci et Z¹, Z², Z³ et Z⁴ sont tels que définis dans l'une quelconque des revendications 1 à 12, à la condition que ce composé ne soit pas un composé de formule 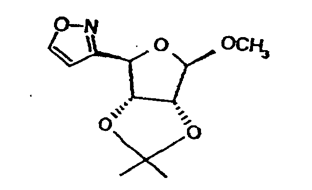 ou un composé de formule : 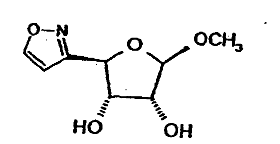 ou un composé de formule :

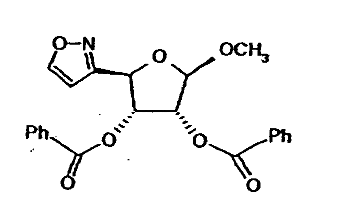

21. Composé de formule (V) : 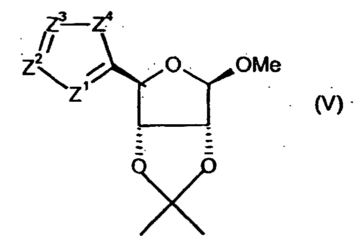 dans laquelle Z¹, Z², Z³ et Z⁴ sont tels que définis dans l'une quelconque des revendications 1 à 12, à la condition que ce composé ne soit pas un composé de formule :

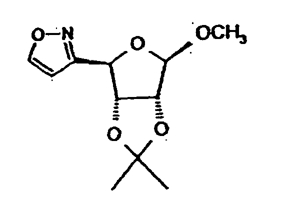

22. Composé de formule (VI) : 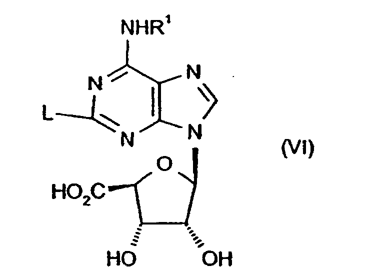 dans laquelle L représente un groupe mobile ou un dérivé protégé de celui-ci et R¹ est tel que défini dans l'une quelconque des revendications 1 à 12, à la condition que ce composé ne soit pas un composé de formule : 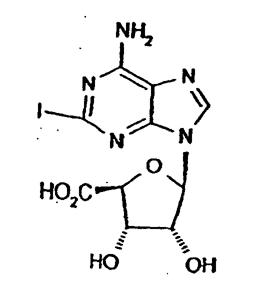 et à la condition que lorsque R¹ représente Ph₂CHCH₂, L ne représente pas un atome de chlore.
